# EUROPEAN PATENT SPECIFICATION

(11) **EP 1 303 258 B1**
(45) Date of publication and mention of the grant of the patent: **11.10.2006**
(21) Application number: 01952071.7
(22) Date of filing: 10.07.2001
(51) Int. Cl.: A61K 9/12, A61K 9/72, A61K 47/24

(54) **NOVEL AEROSOL FORMULATION CONTAINING A POLAR FLUORINATED MOLECULE**
NEUE AEROSOLFORMULIERUNGEN EIN POLARES FLUORINIERTES MOLEKÜL ENTHALTEND
NOUVELLE PREPARATION EN AEROSOL CONTENANT UNE MOLECULE FLUOREE POLAIRE

(30) Priority: 11.07.2000 GB 0016876
(43) Date of publication of application: 23.04.2003
(73) Proprietor: AstraZeneca AB, 151 85 Södertälje (SE)
(72) Inventor: ROGUEDA, Philippe, AstraZeneca R&D Charnwood, Loughborough LE11 5RH (GB)
(86) International application number: PCT/SE2001/001606
(87) International publication number: WO 2002/003958

(56) References cited:
- WO-A1-91/11173
- US-A- 3 557 294
- US-A- 5 849 265
- US-A- 6 149 892
- EDMOND I. EGER II ET AL.: 'Minimum alveolar anesthetic concentration of fluorinated alkanols in rats: Relevance to theories of narcosis' ANESTH. ANALG. vol. 88, 1999, pages 867 - 876, XP002948159

## Description

The present invention relates to a pharmaceutical aerosol formulation for the administration of a pharmaceutically active substance by inhalation.

Pressurised metered dose inhalers (pMDI's) are known in the art. Long standing problems with pMDI's containing suspension formulations include creaming of the suspension, coarse drug suspension, drug flocculation and adhesion to dispensing device.

It has now surprisingly been found that these problems can be overcome with a novel pharmaceutical formulation containing a polar fluorinated molecule in conjunction with a suitable excipient. The formulations of the invention give rise to improved aerosol drug suspension characteristics, i.e. increase of phase separation times (creaming or sedimentation), production of a finer suspension, reduction of particles adhesion to the can walls and inhibition of particle flocculation.

In a first aspect the invention therefore provides a pharmaceutical formulation comprising a drug, an aerosol propellant, a polar fluorinated molecule and an excipient soluble in the polar fluorinated molecule.

Suitable drugs which can be used in the formulation of the invention include all drugs that can be administered via the inhalation route, for example steroids, peptides, oligonucleotides, small organic moecules etc., in particular those administered via a pMDI. Such drugs, which are not limited to those for treating respiratory diseases, include those suitable for administration by nasal delivery and nebulised delivery.

In preferred embodiements, the invention provides stable dispersion for the pulmonary or nasal delivery of one or more bioactive molecules, for local or systemic administration, comprising a fluorinated molecule and an excipient in a propellant or propellant mixture.

The biocative agent may be selected from any therapeutic or diagnostic agent. For example it may be from the group of antiallergics, bronchodilators, bronchoconsitrictors, pulmonary lung surfactants, analgesics, antibiotics, leukotrine inhibitors or antagonists, anticholinergics, mast cell inhibitors, antihistamines, antiinflammatories, antineoplastics, anaesthetics, anti-tuberculars, imaging agents, cardiovascular agents, enzymes, steroids, genetic material, viral vectors, antisense agents, proteins, peptides and combinations thereof.

Examples of specific drugs which can be formulated according to the invention include mometasone, ipratropium bromide, tiotropium and salts thereof, salemeterol, fluticasone propionate, beclomethasone dipropionate, reproterol, clenbuterol, rofleponide and salts, nedocromil, sodium cromoglycate, flunisolide, budesonide, formoterol fumarate dihydrate, Symbicort^{™} (budesonide and formoterol), Viozan^{™}, 3-[2-(4-hydroxy-2-oxo-3H-1,3-benzothiazol-7-yl)ethylamino]-N-[2-[2-(4-methylphenyl)ethoxy)ethyl]propansulphonamide, terbutaline, terbutaline sulphate, salbutamol base and sulphate, fenoterol, 3-[2-(4-Hydroxy-2-oxo-3H-1,3-benzothiazol-7-yl)ethylamino]-N-[2-[2-(4-methylphenyl)ethoxy]ethyl]propanesulphonamide, hydrochloride. All of the above compounds can be in free base form or as pharmaceutically acceptable salts as known in the art.

Suitable aerosol propellants include those known in the art such as hydrofluoroalkane propellants including 1,1,1,2-tetrafluorethane (P134a) or 1,1,1,2,3,3,3-heptafluoropropane (P227). Preferred propellants include P134a or P227 or a mixture of P134a and P227, in particular a density-matched mixture of the two.

Suitable polar fluorinated molecules include those commercially available from companies such as Apollo chemicals and Fluorochem. Preferably the polar fluorinated molecules are pharmaceutically acceptable and are non-toxic and non-irritant. Suitable polar fluorinated molecules must be miscible in sufficient quantity in the propellant and to be able to solubilise the excipient. The fluorinated molecules are preferably liquid at room temperature, although solids are also possible. Preferably the polar fluorinated molecules are linear, more preferably with a short carbon chain. Most preferably the polar fluorinated molecules have oxygen functionality, i.e. contain an oxygen containing group including fluorinated alcohols, ethers, carboxylic acid, esters, aldehydes and ketones, amines and their mixtures, and any other fluorinated compounds with oxygen based functional groups.

Suitable examples of polar fluorinated molecules include:
n Butyl Pentafluoropropionate, Ethyl Perfluoro n-Dodecanoate, Fluorinert (FC-75), 2,2,3,3,3 Pentafluoropropyl Methyl Ether, Methyl Perfluorodecanoate, 2H Perfluoro-5,8,11-Trimethyl-3,6,9,12-Tetrafluoropropylether, Fluorad (FC-430), 1,1,2,2, Tetrafluoroethyl 2,2,3,3 Tetrafluoropropylether, 1H,1H,2H,2H Perfluorooctan-1-ol, 4,4,4 Trifluorobutan-1-ol, Fomblin (MF 402), Fomblin (ZDOL), Perfluoroheptanoic Anhydride, Methyl Perfluoro 2,5,8,11-Tetramethyl 3,6,9,12, Tetraoxapentadecanoate, N,N-Diethyl-2,3,3,3 Tetrafluoropropionamide, Ethyl 11H-Perfluoroundecanoate, 1H,1H,2H,3H,3H Perfluoro-1,2-Nonandiol, 1H,1H, Perfluorononan-1-ol,
Aflunox (606, 1406, 2507, 6008, 14013), Allyl Heptafluorobutyrate, Allyl Heptafluoroisopropyl Ether, Allyl 1,1,2,3,3,3-Hexafluoropropyl Ether, Allyl Perfluoroheptanoate, Allyl Perfluorooctanoate, Allyl 1H,1H Perfluorooctyl Ether, Allyl Perfluoropentanoate, 4-Amino-2,2-Difluorobutyric Acid, 2-Amino-3-Fluorobutyric Acid, 4-Amino-2-Fluorobutyric Acid, 2-Amino-4-Iminoheptafluoropent-2-ene, 2-Amino-4,4,4-Trifluorobutyric Acid, 3-Amino-4,4,4-Trifluorobutyric Acid, 1,1-Bis(diethylamino)tetrafluoro-1-Propene, Bis(heptafluoroisopropyl)ketone, Bis(hexafluoroisopropyl)maleate, Bis(hexafluoroisopropyl)itaconate, Bis[2-iodo-3-(perfluorooctyl)propyladipate, Bis(pefluorooetyl)itaconate, Bis(perfluorooctyl)maleate, Bis(2,2,2-trifluoroethyl)itaconate, Bis(2,2,2-trifluoroethyl)maleate, 1H,1H-2,5-Bis(trifluoromethyl)-3,6-Dioxaundecafluorononanol, 3,3-Bis(trifluoromethyl)-3-Hydroxypropionic Acid, 2,2 Bis (trifluoromethyl) Propionic Acid, n-Butyl-1,1,2,2-Tetrafluoroethyl Ether, n-Butyl Trifluoroacetate, tert-Butyl Trifluoroacetate, 1,1,1,5,5,6,6,7,7,7-Decafluoro-2,4-Heptanedione, 1H,1H,6H-Decfluorohexan-1-ol, 2H,3H-Decafluoropentane, Diethyl Difluoromalonate, 2,2-Difluoroethanol, 2,2-Difluoroethyl acetate, 2,2-Difluoroethyalamine, DL-4,4-Difluoroglutamic acid, 2,2-Difluoromalonamide, Difluoromethyl, 2,2,3,3,3-Pentafluoropropyl Ether, Difluoromethyl 2,2,2-Trifluoroethyl Ether, Difluoromethy 2,2,2-Trifluoroethyl Ether, 1,3-Difluoro-2-propanol, Dimethyl, Hexafluoroglutarate, Dimethyl Octafluoroadipate, Dimethyl Perfluoroazelate, Dimethyl Perfluoro-1,10-decanedicarboxylate, Dimethyl Perfluorosebacate, Dimethyl Perfluorosuberate, Dimethyl Tetrafluorosuccinate, Dimethyl 2,2,2-Trifluoropropionyl Carbinol, 4-Ethoxy-1,1,2-Trifluorobut-1-ene, Ethyl 3-Amino-4,4,4-trifluorocrotonate, Ethyl Ethoxymethylene-3-oxo-4,4,4-trifluorobutyrate, Ethyl 4-Fluoro-3-methyl-2-pentenoate, Ethyl 2-Fluoropropionate, Ethyl Heptafluorobutyrate, Ethyl Heptafluorobutyrylacetate, Ethyl 3-Hydroxy-4,4,4-trifluorobutyrate, Ethyl 2-Methyl-3-hydroxy-4,4,4-trifluorobutyrate, Ethyl Pentafluoropropionate, Ethyl Perfluoroheptanoate, Ethyl Perfluoro-n-dodecanoate including all compounds like CnF2n+1CO2CH2CH3, n= 4 to 16 (some H substitution possible in the CF chain, and double bonds), Ethyl Perfluoro-n-dodecanoate, Ethyl 7H-Perfluoroheptanoate, Ethyl Perfluorononanoate, Ethyl 9H-Perfluorononanoate, Ethyl Perfluorooctanoate, Ethyl Perfluoropentanoate, Ethyl 5H-Perfluoropentanoate, Ethyl 11H-Perfluoroundecanoate, Ethyl 1,1,2,2-Terafluoroethyl Ether, Ethyl 4,4,4-Trifluorobutyrate, Ethyl 3-(Trifluoromethyl)crotonate, Ethyl 4,4,4-Trifluoro-3-(trifluoromethyl)crotonate, Fluorinert (FC40, FC430, FC70, FC71, FC72, FC77, FC84, FC87, FC104, FC6001, FC6003), DL-2-Fluoro-3-alanine, 2-Fluoroethanol, D-Erythro-4-Fluoroglutamic Acid, 2-Fluoroethyl Methacrylate, DL-4-Fluoroglutamic Acid, L-Erythro-4-Fluoroglutamic Acid, D-Threo-4-Fluoroglutamic Acid, DL-Threo-4, Fluoroglutamic Acid, L-Threo-4-Fluoroglutamic Acid, DL-Erythro-4-Fluoroflutamine, L-Erythro-4-Fluoroglutamine, DL-Threo-4-Fluoroglutamine, DL-Erythro-4-Fluoroisoglutamine, L-Erythro-4-Fluoroisoglutamine, DL-Threo-4-Fluoroisoglutamine, 3-Fluoro-DL-Norleucine, Flutec (PP1, PP2, PP3, PP9, PP10, PP11, PP25, PP50), Fomblin (M, Y (L-Vac), Y (H-Vac), Z15, MF402, ZDOL), Galden (HT70, HT85, HT90, HT100, HT110, HT135, HT200, HT230, HT250, HT270), 1H,1H Heptafluorobutan-1-ol, 1H,1H-Heptafluorobutyl Acetate, Heptafluorobutyramide, Heptafluorobutyric Acid, Heptafluorobutyric Anhydride, 4,4,5,5,6,6,6-Heptafluorohexanoic Acid, 4,4,5,5,6,6,6-Heptafluorohexan-1-ol, 4,4,5,5,6,6,6-Heptafluorohex-2-en-1-ol, Heptafluorosiopropyl Methyl Ether, 1,1,1,3,5,5,5-Heptafluoropentane-2,4-dione, Heptafluoropenta-2-ol, 2-Heptafluoropropoxy-2,3,3,3-tetrafluoropropan-1-ol, Heptafluoropropyl Methyl Ether, Heptafluoropropyl 1,2,2,2-tetrafluoroethyl Ether, Heptafluoropropyl Trifluorovinyl Ether, 2,2,3,4,4,4-Hexafluorobutan-1-ol, 2,2,3,3,4,4-Hexafluorobutan-1-ol, 2,2,3,4,4,4, Hexafluorobutyl Difluoromethyl Ether, 2,2,3,4,4,4-Hexafluorobutyl Methacrylate, Hexafluoroglutaramide, Hexafluoroglutaric Acid, Hexafluoroisopropanol, 1,1,1,3,3,3-Hexafluoroisopropyl Acrylate, mono-Hexafluoroisopropyl Itaconate, mono-Hexafluoroisopropyl Maleate, 1,1,1,3,3,3-Hexafluoroisopropyl methacrylate, Hexafluoroisopropyl Methyl Ether, Hexafluoroisopropylurethane-N-ethyl Methacrylate, Hexafluoroleucine, Hexafluoro-2-methylisopropanol, Hexafluoro-1,5-pentanediol, 3,3,4,5,5,5-Hexafluoropentan-2-ol, 1,1,2,3,3,3-Hexafluoropropyl Ethyl Ether, 1,1,2,3,3,3-Hexafluoropropyl Methyl Ether, 4,4,4,6,6,6-Hexafluoro-4-(trifluoromethyl)hexan-1-ol, 4,5,5,6,6,6-Hexafluoro4-(trifluoromethyl) hex-2-enoic Acid, 4,5,5,6,6,6-Hexafluoro-4-(trifluoromethyl) hex-2-en-1-ol, Hexafluoro-DL-valine, Isopropyl Trifluoroacetate, N, Methylbis(heptafluorobutyramide), Methyl Heptafluorobutyrate, Methyl Heptafluoropropyl Ketone, Methyl 2,2,3,3,4,4-hexafluorobutyrate, Methyl 2-hydroxy-2-(trifluoromethyl)pen-4-enoate, N-Methyl-N, methoxytrifluoroacetamide, Methyl Nonafluorobutyl Ether, Methyl Nonafluorobutyl Ketone, Methyl 2,2,3,3,4,4,5,5-octafluoropentanoate, Methyl Pentafluorobut-3-enoate, Methyl Pentafluoropropionate, Methyl Pentafluoropropionylacetate, Methyl Perfluorodecanoate, Methyl Perfluorododecanoate, Methyl Perfluoroheptanoate, Methyl 7H-Perfluoroheptanoate, Methy Perfluorohexadecanoate, Methyl Perfluoro(2-methyl-3-oxahexanoate), Methyl Perfluorononanoate, Methyl Perfluorooctadecanoate, Methyl Perfluoropentadecanoate, Methyl Perfluorotetradecanoate, Methyl Perfluoro-2,5,8,11-tetramethyl-3,6,9,12-tetraoxapentadecanoate, Methyl Perfluorotridecanoate, Methyl Perfluoroundecanoate, Methyl 2,3,3,3-Tetrafluoropropionate, Methyl Trifluoroacetate, Methyl 4,4,4-trifluoroacetoacetate, 2-Methyl-4,4,4-trifluorobutanol, Methyl 4,4,4,-trifluorocrotonate, Methyl 2-(trifluoromethyl), 3,3,3-trifluoropropionate, Methyl Trifluoropropenoate, Methyl Trifluoropyruvate, (Nonafluoro-n-butyl)epoxide, 2-(Nonafluorobutyl)ethyl acrylate, 2-(Nonafluorobutyl)ethyl methacrylate, 6-(nonafluorobutyl)hexanol, 3-(Nonafluorobutyl)-2-hydroxypropyl Acrylate, 3-(Nonafluoro-n-butyl)prop-2-enol, 3-(Nonafluoro-n-butyl)1,2,-propenoxide, 1H,1H,2H,2H-Nonafluorohexan-1-ol, 1H,1H-Nonafluoropentan-1-ol, 2,2,3,3,4,4,5,5-Octafluoro-1,6-hexanediol, 2,2,3,3,4,4,5,5-Octafluorohexane-1,6-diacrylate, 2,2,3,3,4,4,5,5, Octafluorohexane-1,6-diamethacrylate, 3,3,4,4,5,5,6,6-Octafluoro-1,8-octanediol, 1H,1H,1H-Octafluoropenta-1-ol, 2,2,3,3,4,4,5,5 Octofluoro-1,6-hexanediol, 1,1,1,2,2-Pentafluorobutan-2-ol, 1,1,1,2,2-Pentafluoro-6,6-dimethyl-3,5-heptadione, 6-(Pentafluoroethyl)hexan-1-ol, 4,4,5,5,5-Pentafluoropentan-1-ol, 2,2,3,3,3-Pentafluoropropan-1-ol, Pentafluoropropionaldehyde Hydrate, Pentafluoropropionaldehyde Methyl Hemiacetal, Pentafluoropropionamide, 2,2,3,3,3-Pentafluoropropyl Acrylate, 2,2,3,3,3-Pentafluoropropyl Methacrylate, 2,2,3,3,3-Pentafluoropropyl Methyl Ether, 2,2,3,3,3-Pentafluoropropyl 1,1,2,2-Tetrafluoroethyl Ether, 1H,1H,10H,10H-Perfluoro-1,10-decanediol, 1H,1H-Perfluorodecan-1-ol, 1H,1H,2H,2H-Perfluorodecan-1-ol, 1H,1H,2H,2H-Perfluorodecanethiol, 1H,1H,2H,2H-Perfluorodecyl Acrylate, 1H,1H,2H,2H-Perfluorodecyl Methacrylate, 3-(Perfluoro-n-decyl)prop-2-enol, 3-(Perfluoro-n-decyl)-1,2-propenoxide, 1H,1H-Perfluoro-(3,7-dimethyloctan-1-ol), 2H-Perfluoro-(5,8-dimethyl-3,6,9-trioxadodecane), 1H,1H,12H,12H-perfluoro-1,12-dodecanediol, 1H,1H-Perfluorododecan-1-ol, 1H,1H,2H,2H-Perfluorododecan-1-ol, 1H,1H,2H,2H-Perfluorododecyl Acrylate, 1H,1H,2H,2H-Perfluorododecyl Methacrylate, 7H-Perfluoroheptanal, 7H-Perfluoro-1,1-heptanediol, Perfluoroheptanoic Anhydride, 1H,1H-Perfluoroheptan-1-ol, 1H,1H,7H-Perfluoroheptan-1-ol, Perfluoroheptoxypoly(propyloxy) Acrylate, Perfluoroheptoxypoly(propyloxy) Methacrylate, 1H,1H,7H-Perfluoroheptyl Methacrylate, 1H,1H-Perfluorohexadecan-1-ol, 3 Perfluorohexy-2-Hydroxypropyl Methacrylate, 2-(Perfluoro-n-hexyl)acetaldehyde Dimethyl Acetal, 3-Perfluorohexyl-2-hydroxypropyl Acrylate, 3-Perfluorohexyl-2-hydroxypropyl Methacrylate, 3-(Perfluorohexyl)propan-1-ol, 3-(Perfluoro-n-hexyl)prop-2-enol, 3-(Perfluoro-n-hexyl)-1,2-propenoxide, 11-(Perfluoro-n-hexyl)undecanol, 11-(Perfluoro-n-hexyl)undec-10-enol, 6, (Perfluorosiopropyl)hexan-1-ol, 3-(Perfluoro-3-methylbutyl)-2-hydroxy Propyl Acrylate, 3-(Perfluoro-3-methylbutyl)-2-hydroxy Propyl Methacrylate, 1H,1H,2H,2H-Perfluoro-9-methyldecan-1-ol, 2-(Perfluoro-9-methyldecyl)ethyl Acrylate, 2H-perfluoro-5-methyl-3,6-dioxanonane, 1H,1H,2H,2H-Perfluoro-11-methyldodecan-1-ol, Perfluoro-(2-methylhept-3-ene-5-one), 1H,1H,2H,2H, Perfluoro-5-methylhexan-1-ol, 2-(Perfluoro-5-methylhexyl)ethyl Acrylate, 2 (perfluoro-5-methylhexyl)ethyl Methacrylate3-(Perfluoro-5-methylhexyl)-2-hydroxypropyl Acrylate, 3-(Perfluor-5-methylhexyl)-2-hydroxypropyl Methacrylate, 1H,1H,2H,2H,-Perfluoro-7-methylocatn-1-ol, 2-(Perfluoro-7-methyloctyl)ethyl Acrylate, 2-(Perfluoro-7-methyloctyl)ethyl Methacrylate, 6-(Perfluoro-7-methyloctyl)hexanol, 3-(Perfluoro-7-methyloctyl)-2-hydroxypropyl Acrylate, 3-(Perfluoro-7-methyloctyl)-2-hydroxypropyl Methacrylate, 1H,1H,2H,3H,3H-Perfluoro-1,2-nonanediol, 1H,1H,9H,9H-Perfluoro-1,9-nonanediol, 1H,1H-Perfluorononan-1-ol, 1H,1H,9H-perfluorononan-1-ol, 1H,1H,9H-Perfluoronon-1-ene, 1H,1H,9H-Perfluorononyl Acrylate, 1H,2H,9H-Perfluorononyl Methacrylate, 1H,1H-Perfluorooctadecan-1-ol, 1H,1H,8H,8H-Perfluoro-1,8-octanediol, n-Perfluoroctanoic acid Ammonium Salt, 1H,1H-Perfluorooctan-1-ol, 1H,1H,2H,2H-Perfluorooctan-1-ol, 1H,1H,8H-Perfluorooctan-1-ol, Perfluorooctoxy-poly(isobutoxy)-2-chloropropoxy-1,2-propyl Diacrylate, 2-(Perfluoro-n-octyl)acetaldehyde, 2-(Perfluoro-n, octyl)acetaldehyde Diethyl Acetate, Perfluorooctyl Acrylate, 1H,1H-Perfluorooctyl Acrylate, 1H,1H,2H,2H-Perfluorooctyl Acrylate, 6-(Perfluorooctyl)hexanol, 3-(Perfluorooctyl)-2-hydroxypropyl Acrylate, 3-(Perfluorooctyl)-2-hydroxypropyl Methacrylate, mono-Perfluorooctyl Itaconate, mono-Perfluorooctyl Maleate, Perfluorooctyl Methacrylate, 1H,1H-Perfluorooctyl Methacrylate, 3-(Perfluorooctyl)propanol, 3-(Perfluorooctyl)prop-2-enol, 11-(Pefluoro-n-octyl)undec-10-en-1-ol, 1H,1H,5H,5H-Perfluoropentyl-1,5-dimethacrylate, Pefluoropolyether linear & PFO-XR75, Perfluorosebacic Acid, 1H,1H-Perfluorotetradecan-1-ol, 1H,1H,13H-Perfluorotridecan-1-ol, Perfluoro-2-trifluoromethyl-4-oxanonane, Perfluoro-(3,5,5-trimethylhexanoic)acid, 1H,1H-Perfluoro(3,5,5-trimethylhexan-1-ol), 2H-Perfluoro-(5,8,11-trimethyl-3,6,9,12-tetraoxatetradecane), 1H,1H,2H,3H,3H-Perfluoro-1,2,-undecanediol, Perfluoroundecanoic Acid, 1H,1H-Perfluoroundecan-1-ol, 1H,1H,11H-Perfluoroundecan-1-ol, 1H,1H,11H-Perfluoroundecyl Acrylate, 1H,1H,11H-Perfluoroundecyl Methacrylate, Polyperfluoroethylene glycol Diacrylate, Polyperfluoroethylene glycol Dimethacrylate, Sodium Heptafluorobutyrate, Sodium Pentafluoropropionate, 2,2,3,3-Tetrafluoro-1,4-butanediacrylate, 2,2,3,3-Tetrafluorol,4,butanedimethacrylate, 1,1,3,3-Tetrafluorodimethyl Ether, 1,1,2,2-Tetrafluoroethyl 2,2,3,3-tetrafluoropropyl Ether, 1,1,2,2, Tetrafluoroethyl 2,2,2-trifluoroethyl Ether, 1122 Tetrafluoroethyl 222 Trifluoroethyl Ether, 1,2,2,2-Tetrafluoroethyl Trifluoromethyl Ether, 4,5,5,5-Tetrafluoro-4-(heptafluoropropoxy)pentanoic Acid, 4,5,5,5-Tetrafluoro-4-(heptafluoropropoxy)pentan-1-ol, Tetrafluorosuccinic acid, 4,5,5,5-Tetrafluoro-4-(trifluoromethoxy)pentan-1-ol, 4,5,5,5-Tetrafluoro-4-(trifluoromethy)pentan-1-ol, 4,5,5,5-Tetrafluoro-4-(trifluoromethyl)pent-2-en-1-ol, N-(N-Trifluoroacetyl-L-cysteinyl)glycine Methyl Ester, DL-3,3,3-Trifluoro-2-alanine, 4,4,4-Trifluorobutan-1-ol, 1,1,1-Trifluorobutan-2-one, 4,4,4-Trifluorobutan-2-one, 4,4,4-Trifluorobut-2-en-1-ol, 1,1,2-Trifluoro-2-chloroethyl 2,2,2-trifluoroethyl ether, 4,4,4-Trifluorocrotonamide, 4,4,4-Trifluoro-3,3-dimethoxybutanol, 2,2,2-Trifluoroethanol, 2,2,2-Trifluoroethyl Butyrate, 1,2,2-Trifluoroethyl Trifluoromethyl Ether, 1,1,1-Trifluoro-2,4-hexanedione, Beta-Trifluoromethylcrotonic Acid, DL-2-(Trifluoromethyl)leucine, DL-2-(Trifluoromethyl)norleucine, DL-2-(Trifluoromethyl)norvaline, 2-(Trifluoromethyl)propan-2-ol, 6,6,6-Trifluoronorleucine, 5,5,5-Trifluoronorvaline, 1,1,1-Trifluoropropan-2-ol, 3,3,3-Trifluoropropan-1-ol, 1,1,1-Trifluoro-2-propyl Acetate, 4,4,4-Trifluoro-3-(trifluoromethyl)butan-1-ol, 2-Allyl Hexafluorosiopropanol, Butyl Difluoroacetate, n-Butyl Pentafluoropropionate, tert-Butyl Pentafluoropropionate, N,N-Diethyl-2,3,3,3-tetrafluoropropionamide, 22 Difluoroethyl Trifluoromethyl Ether, 1-(Ethoxy)nonafluorobutane, 3-Fluoropropan-1-ol, 3H-Heptafluoro-2,2,4,4-tetrahydroxy Pentane, 2,2,3,3,4,4-Hexafluoro-1,5-pentyl Diacrylate, 1,1,2,3,3,3-Hexafluoropropyl 2,2,2-trifluoro Ethyl Ether, Methyl 2,2-Difluoro-3-oxopentanoate, Methyl 2, Methoxytetrafluoropropionate, Methyl Perfluoro-2,5,8,11-tetramethyl-3,6,9,12-tetraoxapentadecanoate, Methyl 3,3,3-Trifluoro-DL-lactate, 3,3,4,4,4-Pentafluorobutan-2-one, Pentafluorodiemethyl Ether, Pentafluoroethyl Methyl Ether, 2,2,3,3,3-Pentafluoropropyl Trifluoromethyl Ether, 2-(Perfluoroalkyl)ethanol, Perfluoroallylfluorosulphate, Perfluoro-2,5,8,11,14,17,20-heptamethyl-3,6,9,12,15,18-hexaoxahenelcosanoyl Fluoride, Mono-Perfluorooctyl Itaconate, 2H-Perfluoro-5,8,11,14,17-pentamethyl-3,6,9,12,15,18-hexaoxahenicosane, Perfluoropolyether Dinitrile, Polyfluoropolyethyleneacrylate, Polyfluoropolyethylenemethacrylate, 2,2,2-Trifluoroethyl Trifluoromethyl Ether, Perflurodecaline, Perfluorooctyl Bromide, di-Chloro-octyl Bromide and 1H,1H,5H Ocrafluoro-1-pentanol.

Preferably the fluorinated polar molecule is n-Butyl Pentafluoropropionate, Ethyl Perfluoro n-Dodecanoate, Fluorinert (FC-75), 2,2,3,3,3 Pentafluoropropyl Methyl Ether, Methyl Perfluorodecanoate, 2H Perfluoro-5,8,11-Trimethyl-3,6,9,12-Tetrafluoropropylether, Fluorad (FC-430), 1,1,2,2, Tetrafluoroethyl 2,2,3,3 Tetrafluoropropylether, 1H,1H,2H,2H Perfluorooctan-1-ol, 4,4,4 Trifluorobutan-1-ol, Fomblin (MF 402), Fomblin (ZDOL), Perfluoroheptanoic Anhydride, Methyl Perfluoro 2,5,8,11-Tetramethyl 3,6,9,12, Tetraoxapentadecanoate, N,N-Diethyl-2,3,3,3 Tetrafluoropropionamide, Ethyl 11H-Perfluoroundecanoate, 1H,1H,2H,3H,3H Perfluoro-1,2-Nonandiol, 1H,1H, Perfluorononan-1-ol or 1,1,2,2 Tetrafluoroethyl 2,2,2 Trifluoroethyl ether.

Even more preferred fluorinated molecules are 1H,1H,2H,2H Perfluorooctan-1-ol and 1,1,2,2 Tetrafluoroethyl 2,2,2 Trifluoroethyl ether.

The excipient for use in the formulation can be a surfactant or a polymer and combinations thereof, copolymers are particularly favoured. The excipient can either be soluble or miscible in the polar fluorinated molecule. Suitable excipients include:

Acrylidone 1005, Crodesta F160, Methoxy PEG Amine, Methoxy PEG carboxymethyl, 4 arms PEG, Cholic acid, MYRJ 52 P, APG-810-XL, APG-1014-XL, Glucopon 215, Glucopon 600, Brij 52, Gum Xanthan, Salicylic Acid, D-Lacotose monohydrate, α Lactose monohydrate, Lecithin egg, Carrageean, Sokalan CO5, Eudragit RLPO, Eudragit RSPO, Eudragit E100, Eudragit S100, Eudragit L100, Poly (DL-lacide coGlycolide), Gantrez S-97 BF, Gantrez AN-119, Gantrez AN-169, Myristic acid, Poly (lactide EO Lactid), Poly (methyl methacrylate-β -ethylene oxide), Lactose, Carboxymethyl cellulose Sodium Salt, 1-O-n-Octyl β D glucopyranoside, AOT DI-CF4H, Dioctyl-sulfosuccinate sodium salt (AOT), Phospholipon 100, Crodesta F10, Crodesta SL 40, APG 3399, Methoxy-PEG-DSPE MW 2000, Methoxy-PEG-DSPE MW 5000, N Dodecyl β D Maltoside, N Octyl β D Glucopyranoside, α cyclodextrin, β cyclodextrin hydrate, β cyclodextrin, Υ cyclodextrin hydrate, Υ cyclodextrin, Υ cyclodextrin hydrate, Deoxycholic acid, Taurocholic acid, D-Mannitol, Poly (Methyl Methacrylate), Montanov 202, Montanov 68 EC, n Dodecyl β D Glucopyranoside, N Decyl β D Glucopyranoside, n Decyl β D Maltopyranoside, Glucamate DOE-120, Glucate SS, Glucamate SSE-20, Glucam DOE-120, Glucam P10, Glucam E20, Glucam P20 disteared, Glucam P20, Glucquat 125, Brij 30, Brij 96, Crodasinic LS 30, Crossential L99, Copolymer VC 713, Copolymer 958, Glucopon 650 EC, α Tocopherol, PVP K30, K25 and Plasdone K-29/32, PEG 600 and 1000, Three-Arm Poly (ethylene glycol),
lactose based compounds (eg Poly (lactide -co glycolide), Lactitol, Lactose, Cellulose based compounds (e.g. Carboxymethylcellulose, Cellulose, Hydroxypropyl cellulose), Faty acids (e.g. Castor oil), PEG and derivatives (e.g. Star PEG), Sugar compounds (e.g. Alkyl polyglucosides, Methyl glucosides, Sucrose esters, such as Berol AG6202, Glucopon chemical range, Montanov 68, Montanov 202, Grilloten LSE87, Crodesta chemical range), Poly(ethylene Oxide) compounds (e.g. Hydroxy terminated Three-Arm Polyethylene oxide, Hydroxy terminated Eight-Arm Polyethylene oxide, Carboxy terminated Eight-Arm Polyethylene Oxide, 4 Arms Star Polyethylene Oxide, Poly(methyl methacrylate b-ethylene oxide), Poly(t-butyl methacrylate -b-ethylene oxide), Poly(lactide-ethylene oxide-lactide triblock copolymer), Ω -Diacrylonyl terminated poly(lactide-ethylene oxide-lactide) triblock copolymer, Poly(lactone-β-ethylene oxide-β-lactone) triblock copolymer, Poly(ethylene oxide-β-caprolactone), Poly(ethylene oxide-β-propylene oxide) also known as PEO-PPO copolymers, Poly(methy methacrylate-β-ethylene oxide) also known as PMMA-PEO copolymers)). Further examples include Citric acid, Dibutyl Sebacate, Edetic acid, Glyceryl monooleate & monostearate, Glycofinol, Crodamol chemical range, Maltitol, Maltodextrin, Triglyceride, Polymethacrylate, Polyosyethylene alkyl ether, Sodium citrate dihydrate, Sorbitol, Mirj and Brij chemical range, Pluronic chemical range, Acrylidone 1005, Fluorinated AOT with different degrees of fluorination, Cholic acid, Copolymer 958, Copolymer VC713, Crossential L99, Crodasinic LS30, AOT Sodium salt, Phospholipon 100H, Salycilic acid, Sokalan CO5, Poly (lactide co glycolide), Poly(ethylene -β- methyl methacrylate), Poly(ethylene -β-2- vinyl pyridine), Poly(ethylene-β-4-vinyl pyridine), Poly(methyl methacrylate -β- sodium acrylate), Poly(methyl methacrylate-β-sodium methacrylate), PEG derivative compounds (Amino acid - PEG, Carboxyl - PEG copolymers, Methoxy PEG amine, Methoxy PEG carboxymethyl, Branched PEG 4 arms, star PEG, PEG-PLA-PEG triblock copolymer etc...), sugar branched cyclodextrins derivatives, PEO cyclodextrins derivatives, and Dendrimer-PEO-Dendrimer triblock-copolymers.

Preferably the excipient is PEG based. Preferred excipients include Methoxy-PEG-DSPE MW 5000, Eudragit E100, Glucamate DOE 120, Methoxy-PEG-DSPE MW 2000, Acrylidone 1005, Crodesta F160, Methoxy PEG Amine, Methoxy PEG carboxymethyl, 4 arms PEG, Cholic acid, MYRJ 52 P, APG-810-XL, APG-1014-XL, Glucopon 215, Glucopon 600, Brij 52, Gum Xanthan, Salicylic Acid, D-Lacotose monohydrate, α Lactose monohydrate, Lecithin egg, Carrageean, Sokalan CO5, Eudragit RLPO, Eudragit RSPO, Eudragit E100, Eudragit S100, Eudragit L100, Poly (DL-lacide coGlycolide), Gantrez S-97 BF, Gantrez AN-119, Gantrez AN-169, Myristic acid, Poly (lactide EO Lactid), Poly (methyl methacrylate-β-ethylene oxide), Lactose, Carboxymethyl cellulose Sodium Salt, 1-O-n-Octyl β D glucopyranoside, AOT DI-CF4H, Dioctyl-sulfosuccinate sodium salt (AOT), Phospholipon 100, Crodesta F10, Crodesta SL 40, APG 3399, Methoxy-PEG-DSPE MW 2000, Methoxy-PEG-DSPE MW 5000, N Dodecyl β D Maltoside, N Octyl β D Glucopyranoside, α cyclodextrin, β cyclodextrin hydrate, β cyclodextrin, gamma cyclodextrin hydrate, gamma cyclodextrin, gamma cyclodextrin hydrate, Deoxycholic acid, Taurocholic acid, D-Mannitol, Poly (Methyl Methacrylate), Montanov 202, Montanov 68 EC, n Dodecyl β D Glucopyranoside, N Decyl β D Glucopyranoside, n Decyl β D Maltopyranoside, Glucamate DOE-120, Glucate SS, Glucamate SSE-20, Glucam DOE-120, Glucam P10, Glucam E20, Glucam P20 disteared, Glucam P20, Glucquat 125, Brij 30, Brij 96, Crodasinic LS 30, Crossential L99, Copolymer VC 713, Copolymer 958, Glucopon 650 EC, α Tocopherol, PVP K30, K25 and Plasdone K-29/32, PEG 600 and 1000, Three-Arm Poly (ethylene glycol).

Most preferably the excipient is Methoxy-PEG-DSPE MW 5000, Eudragit E100, Glucamate DOE 120 or Methoxy-PEG-DSPE MW 2000.

The grades of fluorinated molecules and excipients mentioned herein are purely indicative and do not limit the scope of this invention. Preferably the fluorinated molecules and excipients are pharmaceutically acceptable.

Other ingredients, for example other co-solvents, stabilisers, surfactants, lubricants, excipients, preservatives, buffers, antioxidants, sweeteners, water trapping agents, bulking agents, and taste masking agents may be included in the formulation of the present invention as desired.

The formulation of the present invention may be prepared, for example, by mixing the fluorinated polar molecule with the excipient, then adding the drug powder to the mixture. Propellant is then added to the drug slury, the formulation obtained is then dispensed in aliquots into specified pMDI which is suitable for nasal or pulmonary drug delivery by any known method, for example under pressure (addition of propellant under pressure) or by cold filling (addition of propellant at a temperature below its boiling point). The pharmaceutically active component may be processed in order to obtain a desired particle size distribution or specific surface properties. For example the pharmaceutically active component may be micronised by conventional methods prior to mixing, or the mixture of pharmaceutically active component may be micronised by conventional methods, after mixing.

Suitably the concentration of the fluorinated polar molecule is from 0.0001 to 55 % weight/weight, more preferably from 0.1 to 25%, and most preferably from 0.3 to 15%. The concentaration of excipient is suitably from 0.001 % to 1%, preferably 0.01 to 1%.

The pMDI device for use with the formulation of the present invention preferably comprises a metal can, for example an aluminium can, closed with a suitable metering valve. Plastic and glass cans can also be used. Suitable cans, coated cans such as cans coated with a fluoropolymer, and metering valves are known in the art.

The pharmaceutical formulations of the present invention are useful for the local or systemic treatment of diseases and may be administered for example via the upper and lower respiratory tract, including by the nasal route. As such the present invention also provides the pharmaceutical aerosol formulation as defined herein for use in therapy; the use of the pharmaceutical aerosol formulation for the manufacture of a medicament for the treatment of diseases via the respiratory tract; and a method for the treatment of a patient in need of therapy, comprising administering to said patient a therapeutically effective amount of the pharmaceutical aerosol formulation of the present invention. It is expected that inflammatory diseases in the respiratory tract, for example asthma, rhinitis, COPD, alveolitis, bronchiolitis and bronchitis can be treated using the present pharmaceutical aerosol formulation.

The pharmaceutical formulation of the present invention is also useful for systemic delivery for many other non-respiratory diseases e.g. cancer, pain control, anaesthesia, infection, vaccinations etc.

In a further aspect the invention provides the use of a polar fluorinated molecule in conjunction with an excipient to reduce deposition and creaming of a pharmaceutical aerosol formulation, and to obtain easily a very fine stable suspension comprising a hydrofluoroalkane propellant having dispersed therein drug particulates.

In a further aspect the invention provides a pharmaceutical aerosol as described herein for use in therapy. The invention further provides a method of treatement of a patient in need of therapy comprising administering to said patient a therapeutically effective amount of a pharmaceutical aerosol formulation as described herein. In particular the invention provides a method of treating asthma, rhinitis and COPD.

The invention will now be illustrated in the following, non-limiting, examples.

### Selection of Examples

A series of tests were performed to select novel formulation combinations. To select suitable fluorinated compounds, their solubility or miscibility in propellants HFA 134a and HFA 227 were tested (this is a pre-requisite for the fluorinated additive to play a suitable role in the formulation). Subsequently the solubility of selected excipients was tested in one of the fluorinated liquids (1H,1H,2H,2H Perfluorooctan-1-ol abbreviated as 4HPFOH). Finally 9 excipients (Methoxy-PEG-DSPE MW 2000, Methoxy-PEG-DSPE MW 5000, Glucamate DOE 120, Cholic Acid, APG 3399, AOT DI-HCF₆, 1 O n Octyl β D Glycopyranoside, 4 arms PEG, and Eudragit E100) were tested in the fluorinated liquids that were miscible in the propellant.

The results of this work are reported in the sections below. Adhesion pictures are shown in the Figures.

### 2.1 Miscibility and solubility of Fluorinated molecules in propellants

For a fluorinated compound to be useful in the novel aerosol formulation, it must preferably be fully miscible or soluble in the propellants at the concentration required. This also implies full miscibility in a mixture of the propellants.

The fluorinated chemical was weighed in a clear PET vial. The vial was then crimped, and subsequently pressure filled with one of the propellants until the desired total weight was reached.

The miscibility and solubility in HFA 227 and 134a are listed in Table 1. The values in brackets indicate the concentration at which the test was done. Solutions at concentrations below these limits are therefore monophasic. The concentrations quoted are not upper limits. It is perfectly possible for the fluorinated compounds to be miscible or soluble at higher concentrations. In the case of the Fluorad compound (C=9.09%w/w), the liquid was found to be insoluble at 9.09 %w/w. However this does not exclude that it could be miscible at a lower concentration, and therefore still be useful for the purpose of the invention.

**Table 1: Miscibility or solubility of fluorinated molecules in propellants**

| NAME | Miscibility or solubility | |
|---|---|---|
| | HFA-134a | HFA-227 |
| Ethyl Perfluoro n-Dodecanoate | Yes | Yes |
| | (C<9.24%w/w) | (C<41.15%w/w) |
| Fluorinert(FC-75) | Yes | Yes |
| | (C<55.87%w/w) | (C<50.94%w/w) |
| 2,2,3,3,3 Pentafluoropropyl Methyl Ether | Yes | Yes |
| | (C<42.63%w/w) | (C<33.49%w/w) |
| Methyl Perfluorodecanoate | Yes | Yes |
| | (C<42.63%w/w) | (C<39.40%w/w) |
| 2H Perfluoro-5,8,11-trimethyl-3,6,9,12-tetrafluoropropylether | Yes | Yes |
| | (C<43.49%w/w) | (C<35.36%w/w) |
| Fluorad(FC-430) | No | Yes |
| | (C=9.09%w/w) | (C<10.62%w/w) |
| 1,1,2,2-tetrafluoroethyl 2,2,3,3 tetrafluoropropylether | Yes | Yes |
| | (C<40.30%w/w) | (C<41.72%w/w) |
| 1H,1H,2H,2H Perfluorooctan-1-ol (4HPFOH) . | Yes | Yes (C<5.17%w/w) |
| | (C<7.30%w/w) | |
| 4,4,4 Trifluorobutan-1-ol | Yes | Yes (C<4.63%w/w) |
| | (C<4.43%w/w) | |
| Fomblin MF 402 | Yes | Yes (C<9.93%w/w) |
| | (C<9.96%w/w) | |
| Fomblin ZDOL | Yes | Yes |
| | (C<9.93%w/w) | (C<10.04%w/w), |
| Perfluoroheptanoic anhydride | Yes | Yes (C<9.13%w/w) |
| | (C<9.89%w/w) | |
| Methyl perfluoro 2,5,9,11-Tetramethyl 3,6,9,12 Tetraoxapentadecanoate | Yes | Yes (C<8.90%w/w) |
| | (C<10.37%w/w) | |
| N,N-diethyl-2,3,3,3 tetrafluoropropionamide | Yes | Yes (C<9.2%w/w) |
| | (C<9.96%w/w) | |
| Ethyl 11H-Perfluoroundecanoate | Yes | Yes (C<4.43%w/w) |
| | (C<4.93%w/w) | |
| 1H,1H,2H,3H,3H Perfluoro-1,2-nonandiol | Yes | Yes (C<3.71%w/w) |
| | (C<4.84%w/w) | |
| 1H,1H, Perfluorononan-1-ol | Yes | Yes (C<4.15%w/w) |
| | (C<4.55%w/w) | |
| n-Butyl Pentafluoropropionate | Yes | Yes |
| | (C=11.93%w/w) I | (C=10.96%w/w) |

The molecules listed in the chemicals list that do not appear in the following table did not show a solubility commensurate with the other compounds, therefore have not been included in this example section. However, they could still be used within the scope of this invention at a lower concentration range and cannot be excluded as potential systems.

### 2.2 Solubility of selected excipients in 4HPFOH

The second test carried out was to evaluate the solubility (or miscibility in the case of liquid samples) of some excipients in 4HPFOH.

The excipients were weighed in glass vials with a screw-on plastic cap. 4HPFOH was added at the required concentration, and the vial sealed with Teflon tape and the screw-on cap. The sample was sonicated and heated to quicken the solubilisation of the excipient. The vial was then allowed to cool down. Observations were subsequently made to asses their solubility (see Table 2 for results).

### 2.3 Solubility of a range of excipients in Fluorinated systems

The last test performed to determine a suitable list of excipients was to assess the solubility of some of the previous excipients in the miscible or soluble fluorinated liquids. Methoxy-PEG-DSPE MW 2000, Methoxy-PEG-DSPE MW 5000, Glucamate DOE 120, Eudragit E100, Cholic Acid, APG 3399, DI-HCF6, 1 O n Octyl β D Glycopyranoside, and 4 arms PEG were chosen for this purpose.

The solubilities were determined by weighing the excipient in a glass vial, adding the fluorinated liquid by weight and sealing the vial with Teflon tape and a screw-on cap. The samples were then heated and sonicated to speed up dissolution and allowed to cool down. Visual observations were made on the cold samples. The concentration of the solutions was 1 %w/w (unless otherwise stated). Therefore, compounds that are recorded as insoluble, are effectively insoluble at 1 %w/w, but could have a lower solubility. The choice of the 1 %w/w limit is arbitrary.

The observations on the solubilities are listed in Table 3, 4 and 5 below. Compounds which are soluble can be used as excipients in the novel formulation. For instance in the case of Methoxy-PEG-DSPE MW 2000, 5 fluorinated molecules can be used in conjunction with the excipient at a concentration of at least 1 %w/w, and at lower concentrations for the 3 other fluorinated molecules.

**Table 2: Solubility of selected excipients in 4HPFOH**

| Name | Concentration %w/w | Solubility or miscibility |
|---|---|---|
| Arlacel P135 USA | 1.00 | Yes |
| 4 arms PEG | 1.02 | Yes |
| Brij 30 | 1.06 | Yes |
| Brij 52 | 0.99 | Yes |
| Brij 96 | 1.2 | Yes |
| Cholic acid | 0.11 | Yes |
| Crossential L99 | 1.21 | Yes |
| Deoxycholic acid | 0.90 | Yes |
| DI-CF4H | 0.11 | Yes |
| DI HCF2 | 0.98 | Yes |
| DI HCF6 | 0.95 | Yes |
| Dioctyl-sulfosuccinate sodium salt | 0.096 | Yes |
| Dodecyltrimethyl Ammonium Bromide | 1.00 | Yes |
| Eudragit E100 | 0.99 | Yes |
| Eudragit RSPO | 1.01 | Yes |
| Glucamate DOE-120 | 1.16 | Yes |
| Glucam E20 | 1.24 | Yes |
| Glucam P20 disteared | 1.18 | Yes |
| Glucam P20 | 1.31 | Yes |
| Glucquat 125 | 1.12 | Yes |
| Methoxy PEG Amine | 1 | Yes |
| Methoxy PEG Propionic Acid | 1.02 | Yes |
| Methoxy PEG carboxymethyl | 0.99 | Yes |
| Methoxy-PEG-DSPE MW 2000 | 1.45 | Yes |
| PEG-600 | 1.17 | Yes |
| PEG 1000 | 0.98 | Yes |
| MYRJ 52 P | 0.99 | Yes |
| N Octyl beta D Glucopyranoside | 0.11 | Yes |
| Nonyltrimethyl Ammonium Bromide | 0.96 | Yes |
| PVP K-25 | 0.95 | Yes |
| PVP K-30 | 1 | Yes |
| Plasdone K-29/32 | 1.08 | Yes |
| Three-Arm Poly (ethylene glycol) | 0.99 | Yes |

**Table 3 Solubility of excipients in Fluorinated systems at 1 %w/w**

| | Methoxy-PEG-DSPE MW 2000 | Methoxy-PEG-DSPE MW 5000 | Glucamate DOE 120 |
|---|---|---|---|
| Fluorad | No | **Yes** | **Yes** |
| 1,1,2,2-tetrafluoroethyl 2,2,3,3 tetrafluoropropylether | **Yes** | **Yes** | **Yes** |
| Fomblin MD 402 | **Yes** | **Yes** | **Yes** |
| Fomblin ZDOL | **Yes** | **Yes** | No |
| Perfluoroheptanoic anhydride | No | **Yes** | **Yes** |
| Methyl perfluoro 2,5,9,11-Tetramethyl 3,6,9,12 Tetraoxapentadecanoate | **Yes** | No | **Yes** |
| N,N-diethyl-2,3,3,3 tetrafluoropropionamide | No | No | **Yes** |
| 1,1,2,2 Tetrafluoroethyl 2,2,2 Trifluoroethyl ether | **Yes** | **Yes** | **Yes** |

**Table 4 Solubility of excipients in Fluorinated systems at 1 %w/w, unless otherwise stated**

| | Cholic Acid | APG 3399 | DI-HCF₆ |
|---|---|---|---|
| Fluorad | No | No | **Yes** |
| 1,1,2,2-tetrafluoroethyl 2,2,3,3 tetrafluoropropylether | No | No | No |
| Fomblin MD 402 | No | No | **Yes** |
| *Fomblin ZDOL* | No | No | No |
| Perfluoroheptanoic anhydride | **Yes** | **Ye**s | **Yes** |
| Methyl perfluoro 2,5,9,11-Tetramethyl 3,6,9,12 Tetraoxapentadecanoate | **Yes** | No | **Yes** |
| N,N-diethyl-2,3,3,3 tetrafluoropropionamide | No | No | **Yes** |
| 1,1,2,2 Tetrafluoroethyl 2,2,2 Trifluoroethyl ether | No | No | Yes (0.1 %w/w) |

**Table 5 Solubility of excipients in Fluorinated systems at 1 %w/w**

| | 1 O n Octyl β D Glycopyranoside | 4 arms PEG | Eudragit E100 |
|---|---|---|---|
| Fluorad | No | No | No |
| 1,1,2,2-tetrafluoroethyl 2,2,3,3 tetrafluoropropylether | Not tested | **Yes** | **Yes** |
| Fomblin MD 402 | **Yes** | **Yes** | **Yes** |
| Fomblin ZDOL | **Yes** | **Yes** | **Yes** |
| Perfluoroheptanoic anhydride | **Yes** | **Yes** | **Yes** |
| Methyl perfluoro 2,5,9,11-Tetramethyl 3,6,9,12 Tetraoxapentadecanoate | No | **Yes** | No |
| N,N-diethyl-2,3,3,3 tetrafluoropropionamide | No | **Yes** | **Yes** |
| 1,1,2,2 Tetrafluoroethyl 2,2,2 Trifluoroethyl ether | No | **Yes** | **Yes** |

From these results, it is possible to devise suitable excipient combinations that will form the novel formulation.

### 3 Examples selected

### 3.1 List of examples and controls

At least 29 novel formulations can be counted from the results in the previous tables, and many more can be elaborated from the previous lists of chemicals. The following combinations were especially assessed:
1- Budesonide with Methoxy-PEG-DSPE MW 5000 and 4HPFOH in HFA 227
2- Budesonide with Methoxy-PEG-DSPE MW 5000 and 4HPFOH in HFA 134a
3- Formoterol Fumarate Dihydrate with Methoxy-PEG-DSPE MW 5000 and 4HPFOH in HFA 227
4- Formoterol Fumarate Dihydrate with Methoxy-PEG-DSPE MW 5000 and 4HPFOH in HFA 134a
5- Budesonide with Eudragit E100 and 4HPFOH in HFA 227
6- Budesonide with Glucamate DOE 120 and 4HPFOH in HFA 227
7- Budesonide with Methoxy-PEG-DSPE MW 2000 and 4HPFOH in HFA 227
8- Formoterol Fumarate Dihydrate with Methoxy-PEG-DSPE MW 2000 and 4HPFOH in HFA 227
9- Terbutaline Sulphate with Methoxy-PEG-DSPE MW 2000 and 4HPFOH in HFA 227
10- 3-[2-(4-hydroxy-2-oxo-3H-1,3-benzothiazol-7-yl)ethylamino]-N-[2-[2-(4-methylphenyl)ethoxy)ethyl]propansulphonamide, with Methoxy-PEG-DSPE MW 2000 and 4HPFOH in HFA 227
11-Formoterol Fumarate Dihydrate with Glucamate DOE 120 and 1,1,2,2 Tetrafluoroethyl 2,2,2 Trifluoroethyl ether in HFA 227

A range of control samples were prepared to compare directly with the novel formulations, these were:
1- Formoterol Fumarate Dihydrate in HFA 227
2- Formoterol Fumarate Dihydrate in HFA 134a
3- Formoterol Fumarate Dihydrate with PEG 1000 and PVP K25 in a HFA 227 and 134a mix.
4- Terbutaline Sulphate in HFA 227
5- Terbutaline Sulphate in HFA 134a
6- Terbutaline Sulphate with PEG 600 and PVP K30 in HFA 227
7- Budesonide in HFA 227
8- Budesonide in HFA 134a
9- Budesonide with PEG 1000 and PVP K25 in HFA 227
10-3-[2-(4-hydroxy-2-oxo-3H-1,3-benzothiazol-7-yl)ethylamino]-N-[2-[2-(4-methylphenyl)ethoxy)ethyl]propansulphonamide, in HFA 227
11- 3-[2-(4-hydroxy-2-oxo-3H-1,3-benzothiazol-7-yl)ethylamino]-N-[2-[2-(4-methylphenyl)ethoxy)ethyl]propansulphonamide, in HFA 134a
12- 3-[2-(4-hydroxy-2-oxo-3H-1,3-benzothiazol-7-yl)ethylamino]-N-[2-[2-(4-methylphenyl)ethoxy)ethyl]propansulphonamide, with PEG 600 and PVP K30 in HFA 227

All drug material used was micronised.

### 3.2 Samples preparation

Samples for adhesion and creaming tests were prepared in clear PET vials fitted with a continuous valve. The excipient and fluorinated molecule were mixed and the drug was weighed into the vial. The mixture of fluorinated molecule and excipient was then added to the drug. Once the continuous valve was manually crimped, the propellant was transferred through the valve under pressure to the desired weight. The samples were sonicated for at least 15 minutes, and left to stand for equilibration for up to 12 hours, before observations were made. The samples were then assessed and kept under standard laboratory conditions.

Samples for sizing were prepared in a similar fashion in 12 ml aluminium cans. The cans were then pierced and their content transferred in the measuring cell.

Examples 5 to 11 were prepared at 6 different concentrations to study the influence of the components concentrations.

### 3.3 Samples concentrations

The examples concentrations can be found below.

### Example 1

Budesonide: 0.125 %w/w
Methoxy-PEG-DSPE MW 5000: 0.320 %w/w
4HPFOH: 31.7 %w/w
HFA 227: to 100 %w/w

### Example 2

Budesonide: 0.174 %w/w
Methoxy-PEG-DSPE MW 5000: 0.286 %w/w
4HPFOH: 28.4 %w/w
HFA 134a: to 100 %w/w

### Example 3

Formoterol Fumarate Dihydrate: 0.154%w/w
Methoxy-PEG-DSPE MW 5000: 0.320 %w/w
4HPFOH: 32.2 %w/w
HFA 227: to 100 %w/w

### Example 4

Formoterol Fumarate Dihydrate: 0.220 %w/w
Methoxy-PEG-DSPE MW 5000: 0.317 %w/w
4HPFOH: 31.5 %w/w
HFA 134a: to 100 %w/w

### Example 5

6 suspensions were prepared

| Sample number | Concentration in HFA 227 of: (%w/w) | | |
|---|---|---|---|
| | Budesonide | Eudragit E 100 | 4HPFOH |
| 5.1 | 0.250 | 0.151 | 17.7 |
| 5.2 | 0.245 | 0.055 | 6.38 |
| 5.3 | 0.234 | 0.545 | 11.5 |
| 5.4 | 0.251 | 0.183 | 2.97 |
| 5.5 | 0.264 | 1.28 | 20.8 |
| 5.6 | 0.253 | 1.12 | 11.3 |

### Example 6

6 suspensions were prepared

| Sample number | Concentration in HFA 227 of: (%w/w) | | |
|---|---|---|---|
| | Budesonide | Glucamate DOE-120 | 4HPFOH |
| 6.1 | 0.262 | 0.166 | 18.3 |
| 6.2 | 0.267 | 0.062 | 6.88 |
| 6.3 | 0.255 | 1.12 | 11.3 |
| 6.4 | 0.264 | 1.33 | 21.2 |
| 6.5 | 0.262 | 0.569 | 12.1 |
| 6.6 | 0.256 | 0.192 | 3.05 |

### Example 7

6 suspensions were prepared

| Sample number | Concentration in HFA 227 of: (%w/w) | | |
|---|---|---|---|
| | Budesonide | Methoxy-PEG-DSPE MW 2000 | 4HPFOH |
| 7.1 | 0.239 | 0.193 | 17.1 |
| 7.2 | 0.260 | 0.078 | 6.9 |
| 7.3 | 0.249 | 0.966 | 11.1 |
| 7.4 | 0.25 | 1.13 | 20.0 |
| 7.5 | 0.26 | 0.519 | 12.1 |
| 7.6 | 0.255 | 0.172 | 3.06 |

### Example 8

6 suspensions were prepared

| Sample number | Concentration in HFA 227 of: (%w/w) | | |
|---|---|---|---|
| | Formoterol Fumarate Dihydrate | Methoxy-PEG-DSPE MW 2000 | 4HPFOH |
| 8.1 | 0.017 | 0.174 | 17.3 |
| 8.2 | 0.0174 | 0.069 | 6.85 |
| 8.3 | 0.0169 | 1.04 | 11.9 |
| 8.4 | 0.0174 | 0.171 | 3.04 |
| 8.5 | 0.0172 | 0.521 | 12.0 |
| 8.6 | 0.0176 | 1.19 | 21.1 |

### Example 9

6 suspensions were prepared

| Sample number | Concentration in HFA 227 of: (%w/w) | | |
|---|---|---|---|
| | Terbutaline Sulphate | Methoxy-PEG-DSPE MW 2000 | 4HPFOH |
| 9.1 | 0.282 | 0.165 | 16.4 |
| 9.2 | 0.312 | 0.047 | 4.7 |
| 9.3 | 0.288 | 0.71 | 8.2 |
| 9.4 | 0.299 | 1.154 | 20.9 |
| 9.5 | 0.295 | 0.51 | 11.8 |
| 9.6 | 0.294 | 0.169 | 3.06 |

### Example 10

6 suspensions were prepared

| Sample number | Concentration in HFA 227 of: (%w/w) | | |
|---|---|---|---|
| | 3-[2-(4-hydroxy-2-oxo-3H-1,3-benzothiazol-7-yl)ethylamino]-N-[2-[2-(4-methylphenyl)ethoxy)ethyl] propansulphonamide, | Methoxy-PEG-DSPE MW 2000 | 4HPFOH |
| 10.1 | 0.043 | 0.209 | 18.5 |
| 10.2 | 0.045 | 0.082 | 7.2 |
| 10.5 | 0.043 | 1.021 | 11.7 |
| 10.6 | 0.043 | 1.163 | 20.7 |
| 10.7 | 0.043 | 0.521 | 12.1 |
| 10.8 | 0.042 | 0.170 | 3.03 |

### Example 11

6 suspensions were prepared

| Sample number | Concentration in HFA 227 of: (%w/w) | | |
|---|---|---|---|
| | Fomoterol Fumarate Dihydrate | Glucamate DOE-120 | 1,1,2,2-tetrafluoroethyl-2,2,2-trifluoroethyl ether |
| 11.1 | 0.017 | 0.063 | 6.98 |
| 11.2 | 0.017 | 0.159 | 18.4 |
| 11.3 | 0.016 | 0.198 | 3.16 |
| 11.4 | 0.016 | 0.587 | 12.1 |
| 11.5 | 0.017 | 1.10 | 12.1 |
| 11.6 | 0.017 | 1.3 | 22.2 |

### Control 1

Formoterol Fumarate Dihydrate: 0.0167 %w/w
HFA 227: to 100 %w/w

### Control 2

Formoterol Fumarate Dihydrate: 0.0167 %w/w
HFA 134a: to 100 %w/w

### Control 3

Formoterol Fumarate Dihydrate: 0.0167 %w/w
PEG 1000: 0.1 %w/w
PVP K25: 0.001 %w/w
HFA 227: 25 %w/w
HFA 134a: to 100 %w/w

### Control 4

Terbutaline Sulphate: 0.300 %w/w
HFA 227: to 100 %w/w

### Control 5

Terbutaline Sulphate: 0.3 %w/w
HFA 134a: to 100 %w/w

### Control 6

Terbutaline Sulphate: 0.299 %w/w
PEG 600: 0.03 %w/w
PVP K30: 0.005 %w/w
HFA 227: to 100 %w/w

### Control 7

Budesonide: 0.260 %w/w
HFA 227: to 100 %w/w

### Control 8

Budesonide: 0.259 %w/w
HFA 134a: to 100 %w/w

### Control 9

Budesonide: 0.259 %w/w
PEG 1000: 0.3 %w/w
PVP K25: 0.001 %w/w
HFA 227: to 100 %w/w

### Control 10

3-[2-(4-hydroxy-2-oxo-3H-1,3-benzothiazol-7-yl)ethylamino]-N-[2-[2-(4-methylphenyl)ethoxy)ethyl]propansulphonamide: 0.427 %w/w
HFA 227: to 100 %w/w

### Control 11

3-[2-(4-hydroxy-2-oxo-3H-1,3-benzothiazol-7-yl)ethylamino]-N-[2-[2-(4-methylphenyl)ethoxy)ethyl]propansulphonamide: 0.428 %w/w
HFA 134a: to 100 %w/w

### Control 12

3-[2-(4-hydroxy-2-oxo-3H-1,3-benzothiazol-7-yl)ethylamino]-N-[2-[2-(4-methylphenyl)ethoxy)ethyl]propansulphonamide: 0.428 %w/w
PEG 600: 0.3 %w/w
PVP K30: 0.0025 %w/w
HFA 227: to 100 %w/w

### 4 Assessment of examples

The novel formulation is especially useful to reduce drug adhesion to the can walls, reduce phase separation times and keep the suspension finely dispersed. Therefore 3 tests were performed: assessment of can wall adhesion, evaluation of creaming or sedimenting rates and sizing of the dispersion. The results were compared with the characteristics of the control samples.

Further tests were carried out to quantify the solubility of the drugs in the fluorinated liquids (the example chosen was 4HPFOH) and to check the degradation of the drugs in 4HPFOH.

### 4.1 Assessment of the extent of drug adhesion

The assessment of the adhesion of drugs to the can walls was done visually and recorded with a digital camera. The samples prepared in PET vials were observed after a couple of days storage. Their were shaken to enable re-dispersion of the creamed or sedimented layer. At this stage it is important to note that samples prepared with HFA 227 will tend to cream and can show some drug adhesion, whereas samples prepared with HFA 134a tend to sediment and because of it show very little adhesion in the head space. The PET vials were offset against a black background and in some cases allowed to settle before a picture was taken. The level of drug adhesion can be seen on the ring across the vial. The absence of a ring means no adhesion. Adhesion pictures can be found as Figures for the range of samples prepared. Control samples with reference photographs have been collated as Figures.

The pictures are strong evidence of the benefits of the novel formulation. Two types of drug adhesion can be listed. Firstly, head space adhesion, where the particles are spread in the whole head space area (e.g. control 6). Secondly, adhesion at the propellant-gas interface, which will be referred to as ring adhesion (e.g. example 7.4). In all the controls, both types of adhesion were present. In the novel formulations however, the first kind of adhesion had disappeared in all but cases 5.6, 7.2, 10.2 and 10.6. Even in these cases, its extent was greatly limited. The ring adhesion did exist in some of the examples, but was very faint (e.g. 7.2 and 7.4).

The samples prepared with HFA 134a were on average better than the ones prepared with HFA 227. As mentioned before this is mostly due to the density difference. If the particles are not at the interface, and remain wetted in the liquid, they are not likely to adhere in the head space and form a dry ring or surface coating.

It is also interesting to note that for the 134a samples the novel formulation forms a milky suspension, i.e. a fine suspension, compared to the controls that tend to be coarser (see the grains in controls 2 and 3 for examples). Furthermore, the novel formulations are more stable than the controls as can be seen from the milky appearance of most of the examples. The creaming time for these samples was longer than the time required to set the vial and take the photograph (- couple of minutes). This was not the case in many of the controls.

Budesonide examples 1, 2, 5, 6 and 7 must be compared with controls 7, 8 and 9 (Budesonide samples). For all the examples the novel formulation reduces drastically the amount of drug adhesion to the wall of the can. In all cases, except examples 5.6 and 7.2, there was virtually no drug on the can wall. Even in the case of examples 5.6 and 7.2, the adhesion was much less than in the control samples. There were instances where a small ring of particles was seen on the can wall, but even this was minimal compared to the controls.

Formoterol Fumarate Dihydrate examples 3, 4, 8 and 11 must be compared with controls 1, 2 and 3. Terbutaline Sulphate examples in series 9 must be compared with controls 4, 5 and 6. 3-[2-(4-hydroxy-2-oxo-3H-1,3-benzothiazol-7-yl)ethylamino]-N-[2-[2-(4-methylphenyl)ethoxy)ethyl]propansulphonamide examples in series 10 must be compared with control 10, 11 and 12. All these systems showed drastically improved performance over their respective control samples, similar to that described for Budesonide.

### 4.2 Assessment of the phase separation kinetics of the novel formulation

The phase separation kinetics of the novel formulation was assessed visually and with the OSCAR technique (Optical Suspension Characterisation). The OSCAR technique records the turbidity of a sample at two different heights as a function of time. Samples can be studied in situ, in the clear PET vials.

Photographic pictures of selected samples were taken at regular intervals to provide evidence of the slow phase separation kinetics. Samples prepared in HFA 227 creamed, whereas samples prepared in HFA 134a sedimented due to the density difference between the particles and the propellants.

Drug suspensions with no added stabilisers take a few seconds to a few minutes to be fully destabilised. The novel formulation however takes a much longer time to phase separate. It takes on average a couple of hours to form a separate solid phase layer. This is a significant improvement over the performance of other HFA formulations, and one of the major advantages of this novel formulation.

Examples 1, 2, 3 and 4 were studied with the OSCAR technique. In all 4 cases, the onset of detectable creaming was in excess of half an hour. For example 4, it is in excess of 3 hours. This is beyond the time scale usually observed in other formulations, in particular with the control samples, where creaming happens within a few minutes.

The other examples were studied visually. Pictures were recorded for all samples just after shaking and one hour after shaking. The picture titled "after shaking" are to be understood as pictures taken within one minute to one minute and a half after shaking of the first vial of the series. The systems were stable at one hour, and remained so well beyond that limit, extending to a couple of days in some instances. The control samples however had much reduced stability and on average creamed within half an hour after shaking. The level of instability was dependent on the concentration of additives. All suspensions had improved stability properties in the range of concentrations studied.

### 4.3 Assessment of the fineness of the novel formulation

Selected novel formulations were sized with a Mastersizer X in situ to demonstrate the absence of flocculation. The Mastersizer X is a laser light diffraction sizing apparatus developed by Malvern. A pressure cell assembly was adapted to be able to perform suspension sizing in propellant. Samples were prepared in 12 ml Aluminium cans fitted with a continuous valve, as described before in the creaming and adhesion section. These cans were then pierced and their content transferred in the measuring chamber with a purpose designed can piercer. 4 drugs were studied, Formoterol Fumarate Dihydrate, Budesonide, Terbutaline Sulphate and 3-[2-(4-hydroxy-2-oxo-3H-1,3-benzothiazol-7-yl)ethylamino]-N-[2-[2-(4-methylphenyl)ethoxy)ethyl]propansulphonamide. All drugs were micronised. They were formulated with Methoxy-PEG-DSPE MW 2000 and 4HPFOH in HFA 227. In addition, Formoterol Fumarate Dihydrate was sized in Glucamate DOE-120 and 1,1,2,2-tetrafluoroethyl-2,2,2-trifluoroethyl ether in HFA 227. The results could then be compared with sizing results of the same drugs in reference HFA formulations. The sizing results have been summarised in the tables below.

### Formoterol Fumarate Dihydrate samples

Formoterol Fumarate Dihydrate was sized in 2 examples of the novel formulation. The first one is based on the combination Methoxy-PEG-DSPE MW 2000 and 4HPFOH in HFA 227. The second one is based on the combination Glucamate DOE-120 and 1,1,2,2-tetrafluoroethyl-2,2,2-trifluoroethyl ether in HFA 227. The HFA formulation used as a reference was based on a PEG 1000 and PVP K25 mixture in a HFA 134a and HFA 227 blend. Processing of the sizing data was done using the Mie theory. The refractive indices values necessary in the Mie theory were either known (for the 1^{st} novel formulation) or approximated from the pure propellant values (2^{nd} novel formulation and reference HFA formulation). The experimental concentrations are listed in Table 6.1, and the sizing results in Table 6.2.

**Table 6.1 Concentrations of Formoterol Fumarate Dihydrate (FFD) samples sized with the Mastersizer X**

| 1^{st} Novel Formulation | 2^{nd} Novel Formulation | Reference HFA formulation |
|---|---|---|
| Methoxy-PEG-DSPE | Glucamate | PEG 1000-0.099 %w/w |
| MW 2000 - 0.171 %w/w | DOE-120-1.25 %w/w | PVP K25 - 0.00099 %w/w |
| 4HPFOH - 3.053 %w/w | 1,1,2,2-tetrafluoroethyl-2,2,2-trifluoroethyl | FFD - 0.0167 %w/ |
| FFD - 0.0174 %w/w | | HFA 134a - 75.12 %w/w |
| HFA 227 to 100 %w/w | ether - 21.3 %w/w | HFA 227 - 24.77 %w/w |
| | FFD - 0.049 %w/w | |
| | HFA 227 to 100 %w/w | |

**Table 6.2 Sizing results for the novel formulations and the reference HFA formulation of Formoterol Fumarate Dihydrate (FFD). Dimensions are expressed in µm. Span is [D(v,0.9)-D(v,0.1)]/D(v,0.5).**

| Sample | D(v,0.1) | D(v,0.5) | D(v,0.9) | D[4,3] | D[3,2] | Peaks | Span |
|---|---|---|---|---|---|---|---|
| 1^{st} novel Formulation | 1.27 | 2.2 | 3.52 | 2.35 | 1.93 | 1 | 1.022 |
| 2^{nd} novel Formulation | 0.90 | 2.52 | 5.24 | 2.86 | 1.76 | 1 | 1.725 |
| Reference HFA formulation | 3.99 | 9.95 | 116.2 | 35.5 | 8.49 | 2 | 11.28 |

The sizing results show that micronised FFD formulated in either new formulations has a narrower size distribution than in the reference HFA formulation, and the particles have a smaller average size. This is because in the novel formulation particles can exist as individual particles and not as clusters. Furthermore the novel formulations are monodisperse. This will have some effect on the performance of the pMDI, and it is expected that the ex-valve dose should be finer as well. A finely dispersed suspension is a good indicator of efficient suspending agents. The suspensions are well and truly stabilised by the added excipients.

### Budesonide samples

2 Budesonide formulations were sized. The novel formulation was based on the combination Methoxy-PEG-DSPE MW 2000 and 4HPFOH in HFA 227. The reference sample was prepared with a PEG 1000 and PVP K25 mixture in HFA 227. Processing of the sizing data was done using the Mie theory. The refractive indices values necessary in the Mie theory were either known (novel formulation) or approximated from the pure propellant values (reference formulation). The experimental concentrations are listed in Table 7.1, and the sizing results on Table 7.2.

**Table 7.1 Concentrations of Budesonide samples sized with the Mastersizer X**

| Novel Formulation | Reference HFA formulation |
|---|---|
| Methoxy-PEG-DSPE | PEG 1000 - 0.299 %w/w |
| MW 2000 - 0.173 %w/w | PVP K25 - 0.001 %w/w |
| 4HPFOH - 3.095 %w/w | Budesonide - 0.256 %w/w |
| Budesonide - 0.253 %w/w | |
| HFA 227 to 100 %w/w | HFA 227 to 100 %w/w |

**Table 7.2 Sizing results for the novel formulation and the reference HFA formulation of Budesonide. Dimensions are expressed in µm. Span is [D(v,0.9)- D(v,0.1)]/ D(v,0.5).**

| Sample | D(v,0.1) | D(v,0.5) | D(v,0.9) | D[4,3] | D[3,2] | Peaks | Span |
|---|---|---|---|---|---|---|---|
| Novel Formulation | 0.53 | 2.13 | 3.68 | 2.20 | 1.30 | 1 | 1.479 |
| Refrence HFA formulation | 7.33 | 33.5 | 87.5 | 41.7 | 15.1 | 1 | 2.395 |

As for FFD, the sizing results show that micronised Budesonide formulated in the new formulation has a narrower size distribution than in the reference formulation, the particles have a smaller average size, and the size distribution is monodisperse.

### Terbutaline sulphate samples

2 Terbutaline sulphate samples were sized. The novel formulation was based on the combination Methoxy-PEG-DSPE MW 2000 and 4HPFOH in HFA 227. The reference sample was prepared with a PEG 600 and PVP K30 mixture in HFA 227. Modelisation of the sizing data was done using the Mie theory. The refractive indices values necessary in the Mie theory were either known (novel formulation) or approximated from the pure propellant values (reference formulation). The experimental concentrations are listed in Table 8.1, and the sizing results on Table 8.2.

**Table 8.1 Concentrations of Terbutaline Sulphate samples sized with the Mastersizer X**

| Novel Formulation | Reference HFA formulation |
|---|---|
| Methoxy-PEG-DSPE | PEG 600 - 0.03 %w/w |
| MW 2000 - 0.1743 %w/w | PVP K30 - 0.005 %w/w |
| 4HPFOH - 3.1126 %w/w | Terbutaline |
| Terbutaline | Sulphate - 0.0612 %w/w |
| Sulphate - 0.0831 %w/w | |
| HFA 227 to 100 %w/w | HFA 227 to 100 %w/w |

**Table 8.2 Sizing results for the novel formulation and the reference HFA formulation of Terbutaline Sulphate. Dimensions are expressed in µm. Span is [D(v,0.9)- D(v,0.1)]/D(v,0.5).**

| Sample | D(v,0.1) | D(v,0.5) | D(v,0.9) | D[4,3] | D[3,2] | Peaks | Span |
|---|---|---|---|---|---|---|---|
| Novel Formulation | 1.46 | 3.96 | 2.73 | 4.53 | 2.73 | 1 | 1.696 |
| Refrence HFA formulation | 5.68 | 13.6 | 40.4 | 23.1 | 10.6 | 1 | 2.543 |

As for FFD and Budesonide, the sizing results show that micronised Terbutaline sulphate formulated in the new formulation has a narrower size distribution than in the reference formulation, the particles are centred on a smaller average size, and the size distribution is monodisperse.

### 3-[2-(4-hydroxy-2-oxo-3H-1,3-benzothiazol-7-yl)ethylamino]-N-[2-[2-(4-methylphenyl)ethoxy)ethyl]propansulphonamide samples

Two 3-[2-(4-hydroxy-2-oxo-3H-1,3-benzothiazol-7-yl)ethylamino]-N-[2-[2-(4-methylphenyl)ethoxy)ethyl]propansulphonamide samples were sized. The novel formulation was based on the combination Methoxy-PEG-DSPE MW 2000 and 4HPFOH in HFA 227. The reference sample was prepared with a PEG 600 and PVP K30 mixture in HFA 227. Modelisation of the sizing data was done using the Mie theory. The refractive indices values necessary in the Mie theory were either known (novel formulation) or approximated from the pure propellant values (reference formulation). The experimental concentrations are listed in Table 9.1, and the sizing results on Table 9.2.

**Table 8.1 Concentrations of 3-[2-(4-hydroxy-2-oxo-3H-1,3-benzothiazol-7-yl)ethylamino]-N-[2-[2-(4-methylphenyl)ethoxy)ethyl]propansulphonamide samples sized with the Mastersizer X.**

| Novel Formulation | Reference HFA formulation |
|---|---|
| Methoxy-PEG-DSPE | PEG 600 - 0.2941 %w/w |
| MW 2000 - 0.1743 %w/w | PVP K30 - 0.0025 %w/w |
| 4HPFOH - 3.1126 %w/w | 3-[2-(4-hydroxy-2-oxo-3H-1,3-benzothiazol-7-yl)ethylamino]-N-[2-[2-(4-methylphenyl)ethoxy)-ethyl]propansulphonamide, - 0.1009 %w/w |
| 3-[2-(4-hydroxy-2-oxo-3H-1,3-benzothiazol-7-yl)ethylamino]-N-[2-[2-(4-methylphenyl)ethoxy)-ethyl]propansulphonamide, - 0.0831 %w/w | |
| HFA 227 to 100 %w/w | HFA 227 to 100 %w/w |

**Table 8.2 Sizing results for the novel formulation and the reference HFA formulation of 3-[2-(4-hydroxy-2-oxo-3H-1,3-benzothiazol-7-yl)ethylamino]-N-[2-[2-(4-methylphenyl)ethoxy)ethyl]propansulphonamide. Dimensions are expressed in µm. Span is [D(v,0.9)- D(v,0.1)]/ D(v,0.5).**

| Sample | D(v,0.1) | D(v,0.5) | D(v,0.9) | D[4,3] | D[3,2] | Peaks | Span |
|---|---|---|---|---|---|---|---|
| Novel Formulation | 1.53 | 3.14 | 39.9 | 10.6 | 2.76 | 2 | 12.23 |
| Reference HFA formulation | 5.9 | 22.2 | 136.3 | 42.1 | 12.9 | 2 | 5.860 |

As for FFD, Budesonide and Terbutaline sulphate, the sizing results show that micronised 3-[2-(4-hydroxy-2-oxo-3H-1,3-benzothiazol-7-yl)ethylamino]-N-[2-[2-(4-methylphenyl)ethoxy)ethyl]propansulphonamide formulated in the new formulation has a narrower size distribution than in the reference formulation, the particles have a smaller average size. Although the size distribution has in this case 2 peaks, the peak centred on 90 µm could be due to the approximation of the imaginary part of the medium refractive index, and may not be representative of the sample. It is this shoulder peak that leads to the high span value. Despite this results, the size distribution is still narrower and smaller than in the reference formulation.

### 4.4 Further tests: solubility of drug compounds in the novel formulation

This invention is concerned with the formulation of pMDI suspensions, but does not exclude the possibility of the formulation of a solution. Although most drug compounds are insoluble in the fluorinated systems, in some instances it is possible to solubilise the drug. Solubility tests were carried out on 4 different drugs in 4HPFOH: Formoterol Fumarate Dihydrate, Budesonide, Terbutaline Sulphate and 3-[2-(4-hydroxy-2-oxo-3H-1,3-benzothiazol-7-yl)ethylamino]-N-[2-[2-(4-methylphenyl)ethoxy)ethyl]propansulphonamide.

Drug suspensions were prepared in sealed glass vials by weight. The suspensions were then allowed to rest over a couple of day to reach equilibrium. They were firstly assessed optically, and in the case of a possible solubility by UV-vis spectroscopy. The solutions were then filtered with 0.2 µm PTFE filters and studied by UV-Vis spectroscopy between 280 nm and 350 nm. A range of suspensions were prepared, to be able to reach saturation levels. Calibration curves were then drawn by plotting the absorbance as a function of concentration. The inflexion point at which the slope of the calibration plot changed was taken as the solubility limit. The experiment was carried out at least 3 times for each drug.

Formoterol Fumarate Dihydrate, Terbutaline Sulphate and 3-[2-(4-hydroxy-2-oxo-3H-1,3-benzothiazol-7-yl)ethylamino]-N-[2-[2-(4-methylphenyl)ethoxy)ethyl]-propansulphonamide are insoluble in 4HPFOH. Suspensions of the corresponding drug were observed optically at C= 0.1 ppm(w/w). At this concentration, particles were visible in the bulk of the solution. Their respective solubilities are therefore less than 0.1 ppm(w/w). I.e. the compounds can be considered as insoluble. Budesonide, however, is soluble in 4HPFOH. Its solubility limit measured by UV-Vis Spectroscopy is between 0.219 %w/w and 0.246 %w/w.

### 4.5 Further tests: stability of drug compounds in the novel formulation

The stability of Formoterol Fumarate Dihydrate and Budesonide in 4HPFOH were tested and compared with their stability in ethanol.

4 solutions were prepared in glass vials sealed with Teflon tape: Formoterol in 4HPFOH, Formoterol in ethanol, Budesonide in 4HPFOH and Budesonide in ethanol. The concentrations of Formoterol solutions were 0.792 %w/w in 4HPFOH and 1.365 %w/w in ethanol. The concentrations of Budesonide were 0.9315 %w/w in 4HPFOH and 1.215 %w/w in ethanol.

After 3 weeks storage, the levels of total impurity levels in excess of 0.01 % in the Formoterol solutions were 0.782 % for the ethanol solution, and 0.245 % in 4HPFOH. In the case of Budesonide, the levels of impurities were 0.23 %w/w in ethanol and 0.14 %w/w in 4HPFOH.

The impurities come from the degradation of the drug molecule in pure solvent. The total level of impurities in the fluorinated system was therefore up to 3 times less than in ethanol. Drug compounds are therefore more stable in the novel formulation than in other pMDI formulations that use co-solvents. This is yet an other distinct advantage of this novel formulation.

### Explanation of Figures

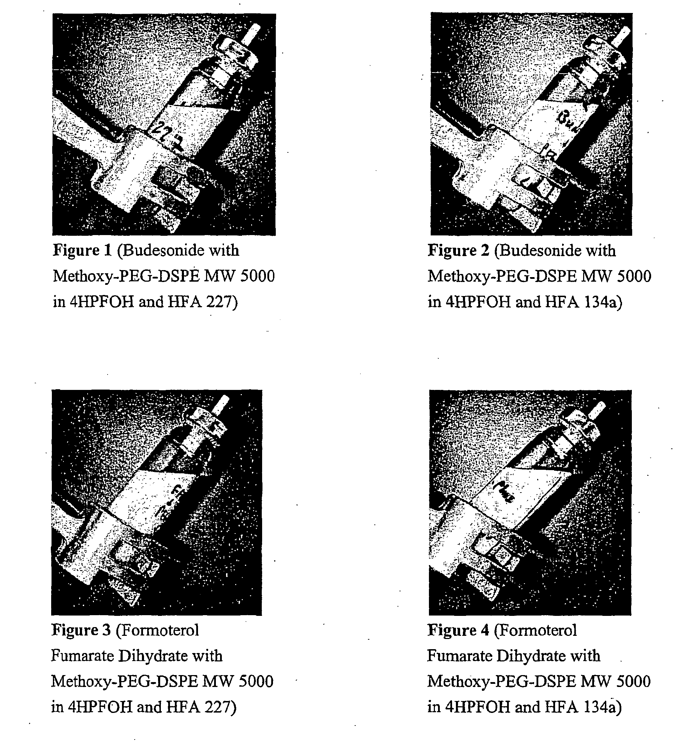

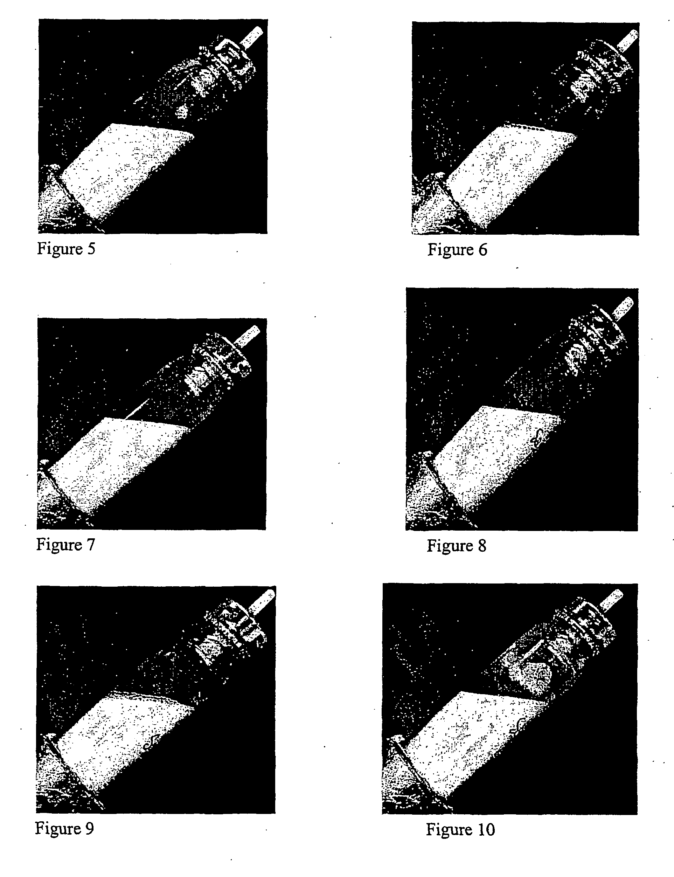

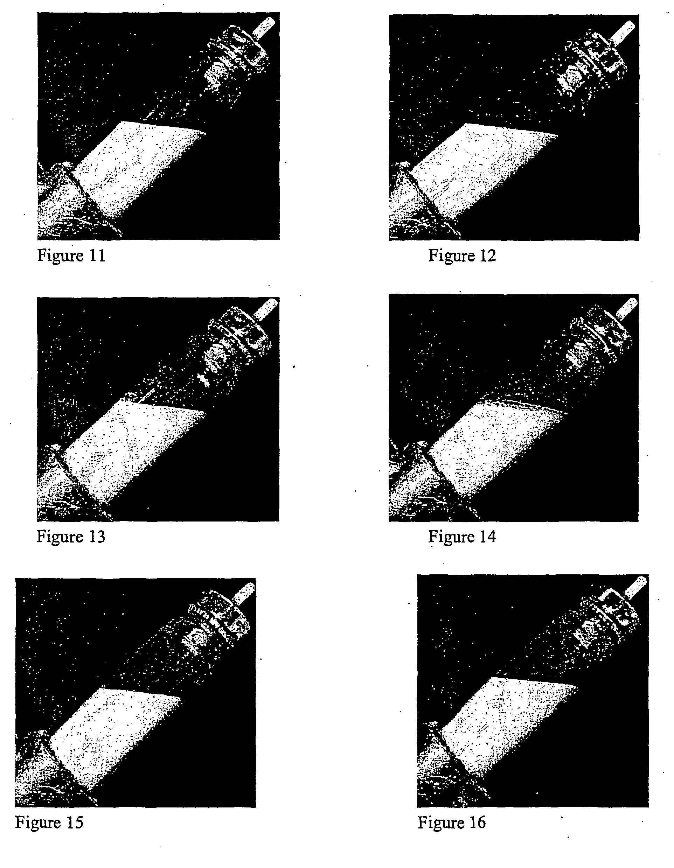

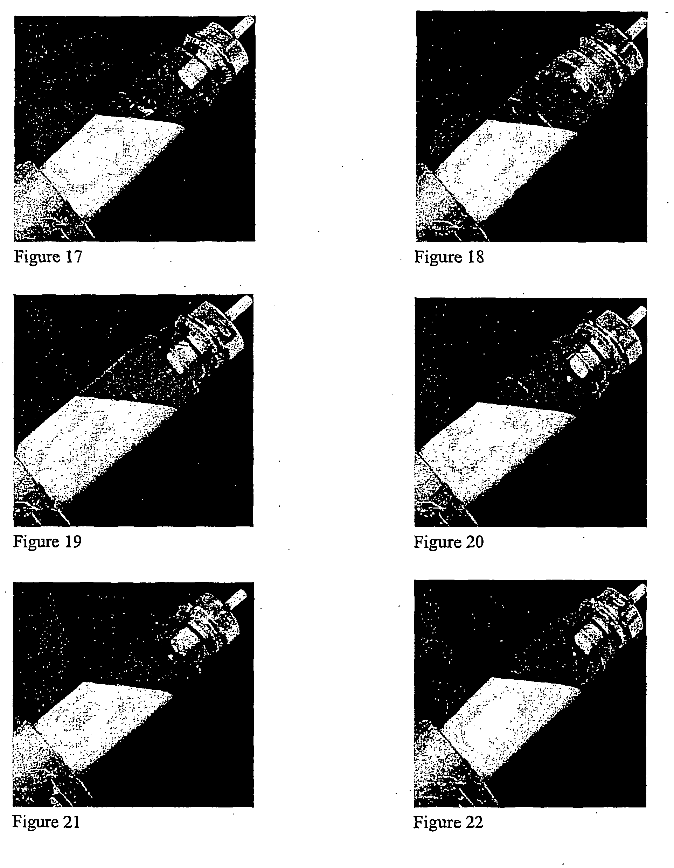

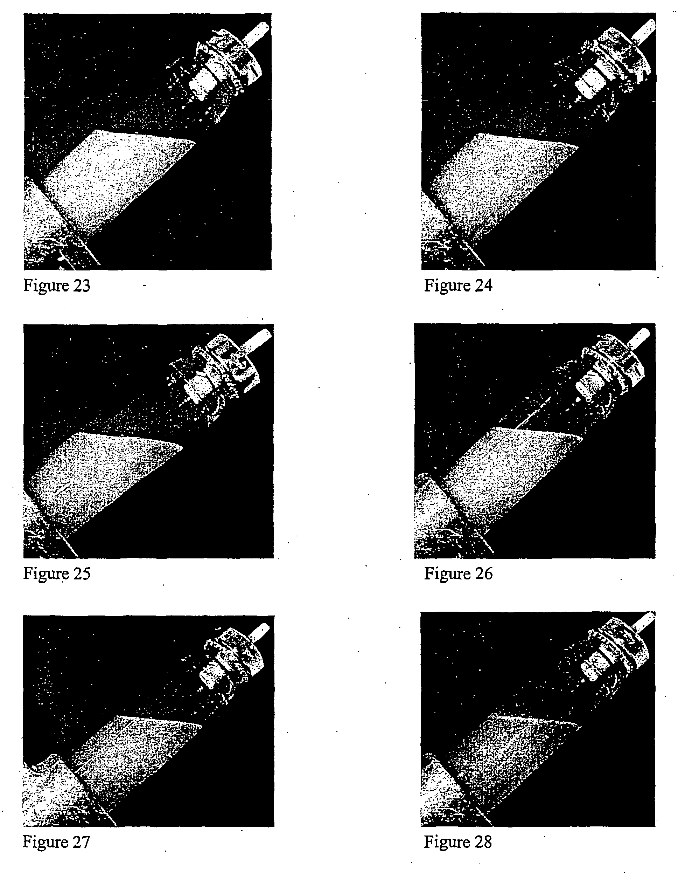

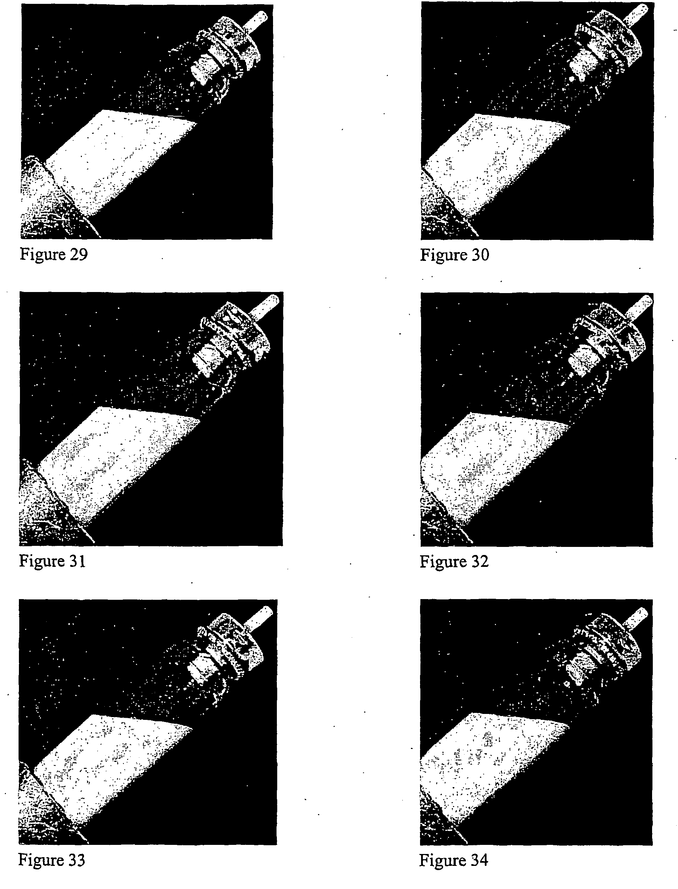

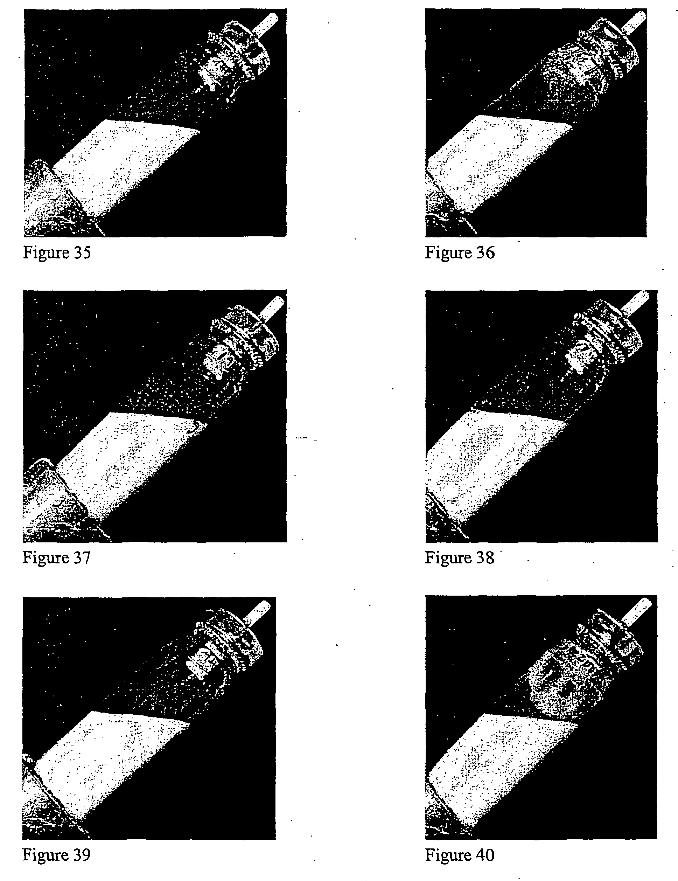

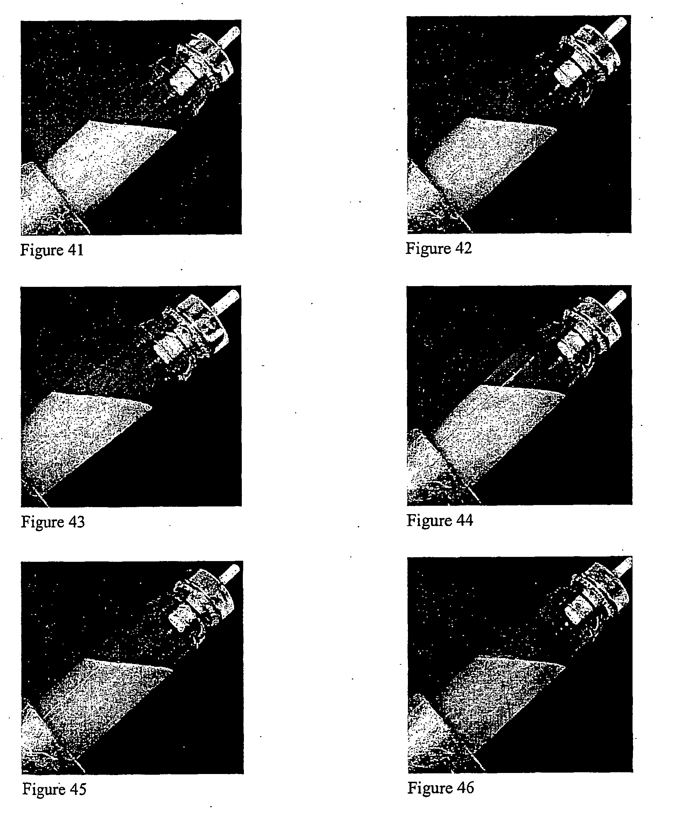

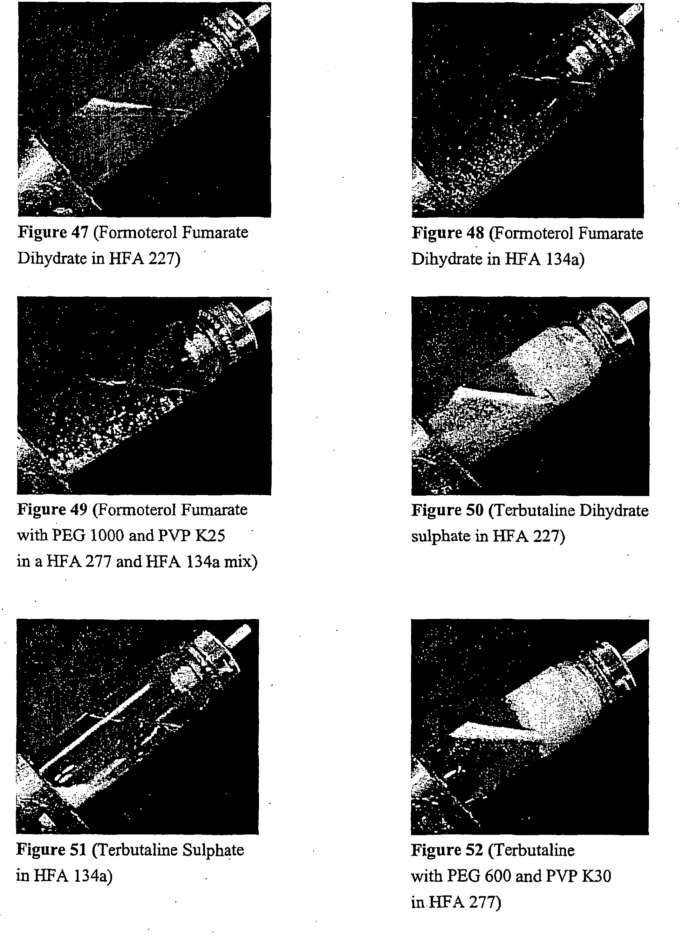

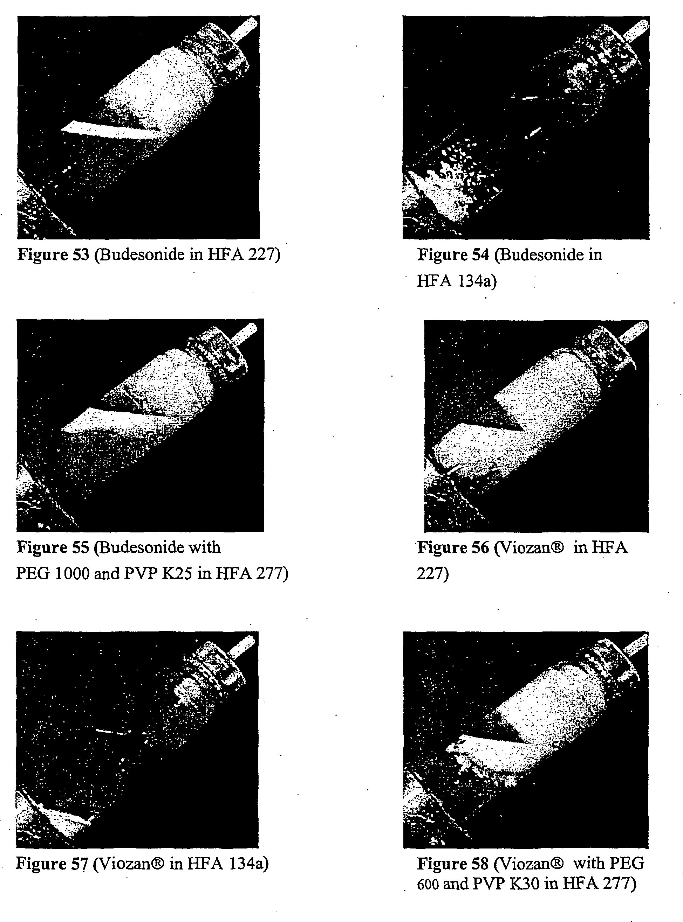

### Figures 1-58 show adhesion pictures for the samples prepared for the examples and controls as follows:

| **Figure** | **Example** | **Figure** | **Example** | **Figure** | **Example** |
|---|---|---|---|---|---|
| 1 | 1 | 21 | 7.5 | 41 | 11.1 |
| 2 | 2 | 22 | 7.6 | 42 | 11.2 |
| 3 | 2 | 23 | 8.1 | 43 | 11.3 |
| 4 | 4 | 24 | 8.2 | 44 | 11.4 |
| 5 | 5.1 | 25 | 8.3 | 45 | 11.5 |
| 6 | 5.2 | 26 | 8.4 | 46 | 11.6 |
| 7 | 5.3 | 27 | 8.5 | **Figure** | **Control** |
| 8 | 5.4 | 28 | 8.6 | 47 | 1 |
| 9 | 5.5 | 29 | 9.1 | 48 | 2 |
| 10 | 5.6 | 30 | 9.2 | 49 | 3 |
| 11 | 6.1 | 31 | 9.3 | 50 | 4 |
| 12 | 6.2 | 32 | 9.4 | 51 | 5 |
| 13 | 6.3 | 33 | 9.5 | 52 | 6 |
| 14 | 6.4 | 34 | 9.6 | 53 | 7 |
| 15 | 6.5 | 35 | 10.1 | 54 | 8 |
| 16 | 6.6 | 36 | 10.2 | 55 | 9 |
| 17 | 7.1 | 37 | 10.5 | 56 | 10 |
| 18 | 7.2 | 38 | 10.6 | 57 | 11 |
| 19 | 7.3 | 39 | 10.7 | 58 | 12 |
| 20 | 7.4 | 40 | 10.8 | | |

## Claims

1. A pharmaceutical formulation comprising a drug, an aerosol propellant, a polar fluorinated molecule which is liquid at room temperature and a PEG excipient soluble in the polar fluorinated molecule, wherein the polar fluorinated molecule is selected from n-Butyl Pentafluoropropionate, Ethyl Perfluoro n-Dodecanoate, Fluorinert (FC-75), 2,2,3,3,3 Pentafluoropropyl Methyl Ether, Methyl Perfluorodecanoate, 2H Perfluoro-5,8,11-Trimethyl-3,6,9,12-Tetrafluoropropylether, Fluorad (FC-430), 1,1,2,2, Tetrafluoroethyl 2,2,3,3 Tetrafluoropropylether, 1H,1H,2H,2H Perfluorooctan-1-ol, 4,4,4 Trifluorobutan-1-ol, Fomblin (MF 402), Fomblin (ZDOL), Perfluoroheptanoic Anhydride, Methyl Perfluoro 2,5,8,11-Tetramethyl 3,6,9,12, Tetraoxapentadecanoate, N,N-Diethyl-2,3,3,3 Tetrafluoropropionamide, Ethyl 11H-Perfluoroundecanoate, 1H,1H,2H,3H,3H Perfluoro-1,2-Nonandiol, 1H,1H, Perfluorononan-1-ol or 1,1,2,2 Tetrafluoroethyl 2,2,2 Trifluoroethyl ether.

2. A pharmaceutical aerosol formulation as claimed in claim 1 wherein the drug is selected from budesonide, formoterol, Symbicort^{™} (budesonide and formoterol), Viozan^{™}, 3-[2-(4-hydroxy-2-oxo-3H-1,3-benzothiazol-7-yl)ethylamino]-N-[2-[2-(4-methylphenyl)ethoxy)ethyl]propansulphonamide, terbutaline, salbutamol base and sulphate, fenoterol, or 3-[2-(4-Hydroxy-2-oxo-3H-1,3-benzothiazol-7-yl)ethylamino]-N-[2-[2-(4-methylphenyl)ethoxy]ethyl]propanesulphonamide and pharmaceutically acceptable salts thereof.

3. A pharmaceutical aerosol formulation as claimed in claim 1 or 2 wherein the propellant is a fluorinated molecule.

4. A pharmaceutical aerosol formulation as claimed in any one of claims 1 to 3 wherein the propellant is HFA 134a or HFA 227 or a mixture of HFA 134a and HFA 227.

5. A pharmaceutical aerosol formulation as claimed in claim 1 to 4 wherein the polar fluorinated molecule is selected from:
is n-Butyl Pentafluoropropionate, Ethyl Perfluoro n-Dodecanoate, Fluorinert (FC-75), 2,2,3,3,3 Pentafluoropropyl Methyl Ether, Methyl Perfluorodecanoate, 2H Perfluoro-5,8,11-Trimethyl-3,6,9,12-Tetrafluoropropylether, Fluorad (FC-430), 1,1,2,2, Tetrafluoroethyl 2,2,3,3 Tetrafluoropropylether, 1H,1H,2H,2H Perfluorooctan-1-ol, 4,4,4 Trifluornbutan-1-ol, Fomblin (MF 402), Fomblin (ZDOL), Perfluoroheptanoic Anhydride, Methyl Perfluoro 2,5,8,11-Tetramethyl 3,6,9,12,Tetraoxapentadecanoate, N,N-Diethyl-2,3,3,3 Tetrafluoropropionamide, Ethyl 11H-Perfluoroundecanoate, 1H,1H,2H,3H,3H Perfluoro-1,2-Nonandiol, 1H,1H, Perfluorononan-1-ol or 1,1,2,2 Tetrafluoroethyl 2,2,2 Trifluoroethyl ether.

6. A pharmaceutical aerosol formulation as claimed in any preceding claim, wherein the excipient is a PEG co-polymer.

7. A pharmaceutical aerosol formulation as claimed in any preceding claim, wherein the excipient is a PEG-phospholipid.

8. A pharmaceutical aerosol formulation as claimed in any preceding claim, wherein the excipient is Methoxy-PEG-DSPE MW 5000, or Methoxy-PEG-DSPE MW 2000.

9. An aerosol can containing a formulation as claimed in any of claims 1 to 8.

10. A can according to claim 9 which is made of metal.

11. An aerosol can as claimed in claim 9 or 10 wherein the internal surfaces of the can are coated with a fluoropolymer.

12. A pharmaceutical aerosol formulation as claimed in any of claims 1 to 8 for use in therapy.

13. A pharmaceutical aerosol formulation as claimed in any of claims 1 to 8 for use in the treatment of asthma, rhinitis or COPD.

## Patentansprüche

1. Pharmazeutische Formulierung, umfassend ein Arzneimittel, ein Aerosol-Treibmittel, ein polares fluoriniertes Molekül, das bei Raumtemperatur flüssig ist, und ein PEG-Hilfsmittel, das in dem polaren fluorinierten Molekül löslich ist, wobei das polare fluorinierte Molekül aus n-Butylpentafluorpropionat, Ethylperfluor-n-dodecanoat, Fluorinert (FC-75), 2,2,3,3,3-Pentafluorpropylmethylether,
Methylperfluordecanoat, 2H-Perfluor-5,8,11-trimethyl-3,6,9,12-tetrafluorpropylether, Fluorad (FC-430), 1,1,2,2-Tetrafluorethyl-2,2,3,3-tetrafluorpropylether, 1H,1H,2H,2H-Perfluoroctan-1-ol, 4,4,4-Trifluorbutan-1-ol, Fomblin (MF 402), Fomblin (ZDOL), Perfluorheptansäureanhydrid, Methylperfluor-2,5,8,11-tetramethyl-3,6,9,12-tetraoxapentadecanoat, N,N-Diethyl-2,3,3,3-tetrafluorpropionamid, Ethyl-11H-perfluorundecanoat, 1H,1H,2H,3H,3H-Perfluor-1,2-nonandiol, 1H,1H-Perfluornonan-1-ol oder 1,1,2,2-Tetrafluorethyl-2,2,2-trifluorethylether ausgewählt ist.

2. Pharmazeutische Aerosolformulierung nach Anspruch 1, wobei das Arzneimittel aus Budesonid, Formoterol, Symbicort^{™} (Budesonid und Formoterol), Viozan^{™}, 3-[2-(4-Hydroxy-2-oxo-3H-1,3-benzothiazol-7-yl)ethylamino]-N-[2-[2-(4-methylphenyl)ethoxy)ethyl]propansulfonamid, Terbutalin, Salbutamolbase und -sulfat, Fenoterol oder 3-[2-(4-Hydroxy-2-oxo-3H-1,3-benzothiazol-7-yl)-ethylamino]-N-[2-[2-(4-methylphenyl)ethoxy]ethyl]-propansulfonamid und pharmazeutisch annehmbaren Salzen davon ausgewählt ist.

3. Pharmazeutische Aerosolformulierung nach Anspruch 1 oder 2, wobei das Treibmittel ein fluoriniertes Molekül ist.

4. Pharmazeutische Aerosolformulierung nach einem der Ansprüche 1 bis 3, wobei das Treibmittel HFA 134a oder HFA 227 oder eine Mischung von HFA 134a und HFA 227 ist.

5. Pharmazeutische Aerosolformulierung nach den Ansprüchen 1 bis 4, wobei das polare fluorinierte Molekül aus n-Butylpentafluorpropionat, Ethylperfluor-n-dodecanoat, Fluorinert (FC-75), 2,2,3,3,3-Pentafluorpropylmethylether, Methylperfluordecanoat, 2H-Perfluor-5,8,11-trimethyl-3,6,9,12-tetrafluorpropylether, Fluorad (FC-430), 1,1,2,2-Tetrafluorethyl-2,2,3,3-tetrafluorpropylether, 1H,1H,2H,2H-Perfluoroctan-1-ol, 4,4,4-Trifluorbutan-1-ol, Fomblin (MF 402), Fomblin (ZDOL), Perfluorheptansäureanhydrid, Methylperfluor-2,5,8,11-tetramethyl-3,6,9,12-tetraoxapentadecanoat, N,N-Diethyl-2,3,3,3-tetrafluorpropionamid, Ethyl-11H-perfluorundecanoat, 1H,1H,2H,3H,3H-Perfluor-1,2-nonandiol, 1H,1H-Perfluornonan-1-ol oder 1,1,2,2-Tetrafluorethyl-2,2,2-trifluorethylether ausgewählt ist.

6. Pharmazeutische Aerosolformulierung nach einem der vorhergehenden Ansprüche, wobei das Hilfsmittel ein PEG-Copolymer ist.

7. Pharmazeutische Aerosolformulierung nach einem der vorhergehenden Ansprüche, wobei das Hilfsmittel ein PEG-Phospholipid ist.

8. Pharmazeutische Aerosolformulierung nach einem der vorhergehenden Ansprüche, wobei das Hilfsmittel Methoxy-PEG-DSPE mit einem Molekulargewicht von 5000 oder Methoxy-PEG-DSPE mit einem Molekulargewicht von 2000 ist.

9. Aerosoldose, enthaltend eine Formulierung nach einem der Ansprüche 1 bis 8.

10. Dose nach Anspruch 9, die aus Metall gefertigt ist.

11. Aerosoldose nach Anspruch 9 oder 10, wobei die Innenseiten der Dose mit einem Fluorpolymer beschichtet sind.

12. Pharmazeutische Aerosolformulierung nach einem der Ansprüche 1 bis 8 zur therapeutischen Verwendung.

13. Pharmazeutische Aerosolformulierung nach einem der Ansprüche 1 bis 8 zur Verwendung zur Behandlung von Asthma, Rhinitis oder COPD.

## Revendications

1. Formulation pharmaceutique comprenant un médicament, un propulseur d' aérosol, une molécule fluorée polaire qui est liquide à la température ambiante et un excipient PEG soluble dans la molécule fluorée polaire, **caractérisée en ce que** la molécule fluorée polaire est choisie parmi le n-Butyl-Pentafluoropropionate, l'Ethyl-Perfluoro-n-Dodécanoate, le Fluorinert(FC-75), le 2,2,3,3,3-Pentafluoropropyl-Méthyléther, le Méthyl-Perfluorodécanoate, le 2H-Perfluoro-5,8,11-Triméthyl-3,6,9,12-Tétrafluoropropyléther, le Fluorad (FC-430), le 1,1,2,2-Tétrafluoroéthyl-2,2,3,3-Tétrafluoropropyléther, le 1H, 1H, 2H, 2H-Perfluorooctan-1-ol, le 4,4,4-Trifluorobutan-1-ol, la Fombline (MF 402), la Fombline (ZDOL), l'Anhydride Perfluoroheptanoïque, le Méthyl-Perfluoro-2,5,8,11-Tétraméthyl-3,6,9,12-Tétraoxapentadécanoate, le N,N-Diéthyl-2,3,3,3-Tétrafluoropropionamide, l'Ethyl-11H-Perfluoroundécanoate, le 1H, 1H, 2H, 3H, 3H-Perfluoro-1,2-Nonandiol, le 1H,1H-Perfluorononan-1-ol ou le 1,1,2,2-Tétrafluoroéthyl-2,2,2-Trifluoroéthyléther.

2. Formulation d'aérosol pharmaceutique selon la revendication 1, **caractérisée en ce que** le médicament est choisi parmi le budésonide, le formotérol, le Symbicort^{™} (budésonide et formotérol), le Viozan^{™}, le 3-[2-(4-Hydroxy-2-oxo-3H-1,3-benzothiazol-7-yl)éthylamino]-N-[2-[2-(4-méthylphényl)éthoxy)éthyl]propanesulfonamide, la terbutaline, le salbutamol base et sulfate, le fénotérol ou le 3-[2-(4-Hydroxy-2-oxo-3H-1,3-benzothiazol-7-yl)éthylamino]-N-[2-[2-(4-méthylphényl)éthoxy]éthyl]propanesulfonamide et les sels pharmaceutiquement acceptables de ceux-ci.

3. Formulation d'aérosol pharmaceutique selon la revendication 1 ou 2, **caractérisée en ce que** le propulseur est une molécule fluorée.

4. Formulation d'aérosol pharmaceutique selon l'une quelconque des revendications 1 à 3, **caractérisée en ce que** le propulseur est l'HFA 134a ou l'HFA 227 ou un mélange d'HFA 134a et d'HFA 227.

5. Formulation d'aérosol pharmaceutique selon les revendications 1 à 4, **caractérisée en ce que** la molécule fluorée polaire est choisie parmi : le n-Butyl-Pentafluoropropionate, l'Ethyl-Perfluoro-n-Dodécanoate, le Fluorinert(FC-75), le 2,2,3,3,3-Pentafluoropropyl-Méthyléther, le Méthyl-Perfluorodécanoate, le 2H-Perfluoro-5,8,11-Triméthyl-3,6,9,12-Tétrafluoropropyléther, le Fluorad (FC-430), le 1,1,2,2-Tétrafluoroéthyl-2,2,3,3-Tétrafluoropropyléther, le 1H,1H,2H,2H-Perfluorooctan-1-ol, le 4,4,4-Trifluorobutan-1-ol, la Fombline (MF 402), la Fombline (ZDOL), l'Anhydride Perfluoroheptanoïque, le Méthyl-Perfluoro-2,5,8,11-Tétraméthyl-3,6,9,12-Tétraoxapentadécanoate, le N,N-Diéthyl-2,3,3,3-Tétrafluoropropionamide, l'Ethyl-11H-Perfluoroundécanoate, le 1H,1H,2H,3H,3H-Perfluoro-1,2-Nonandiol, le 1H,1H-Perfluorononan-1-ol ou le 1,1,2,2-Tétrafluoroéthyl-2,2,2-Trifluoroéthyléther.

6. Formulation d'aérosol pharmaceutique selon l'une quelconque des revendications précédentes, **caractérisée en ce que** l'excipient est un copolymère de PEG.

7. Formulation d'aérosol pharmaceutique selon l'une quelconque des revendications précédentes, **caractérisée en ce que** l'excipient est un PEG-phospholipide.

8. Formulation d'aérosol pharmaceutique selon l'une quelconque des revendications précédentes, **caractérisée en ce que** l'excipient est le Méthoxy-PEG-DSPE PM 5000 ou le Méthoxy-PEG-DSPE PM 2000.

9. Bombe aérosol contenant une formulation selon l'une quelconque des revendications 1 à 8.

10. Bombe selon la revendication 9, **caractérisée en ce qu'**elle est en métal.

11. Bombe aérosol selon la revendication 9 ou 10, **caractérisée en ce que** les surfaces internes de la bombe sont revêtues d'un fluoropolymère.

12. Formulation d'aérosol pharmaceutique selon l'une quelconque des revendications 1 à 8, destinée à être utilisée en thérapie.

13. Formulation d'aérosol pharmaceutique selon l'une quelconque des revendications 1 à 8, destinée à être utilisée dans le traitement de l'asthme, de la rhinite et de BPCO.
